(19) Europäisches Patentamt
European Patent Office
Office européen des brevets

(11) EP 1 973 937 B1

(12) EUROPEAN PATENT SPECIFICATION

(45) Date of publication and mention
of the grant of the patent:
**25.05.2011 Bulletin 2011/21**

(51) Int Cl.:
*C07K 14/435* (2006.01)          *C12N 15/12* (2006.01)
*G01N 33/68* (2006.01)

(21) Application number: 07706220.6

(22) Date of filing: **10.01.2007**

(86) International application number:
**PCT/IT2007/000020**

(87) International publication number:
**WO 2007/080621 (19.07.2007 Gazette 2007/29)**

(54) **LUMINESCENT TRANSGENIC NON HUMAN ANIMALS, PROGENY, CELL DERIVATIVES AND USES THEREOF**

TRANSGENE NICHTMENSCHLICHE LEUCHTTIERE, ABKÖMMLING, ZELLDERIVATE UND VERWENDUNG

ANIMAUX TRANSGÉNIQUES NON HUMAINS LUMINESCENTS, LEUR PROGÉNITURE, LEURS DÉRIVÉS CELLULAIRES ET LEURS APPLICATIONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.01.2006 EP 06000452**
**27.10.2006 EP 06022458**

(43) Date of publication of application:
**01.10.2008 Bulletin 2008/40**

(73) Proprietor: **Axxam S.P.A.**
**20132 Milano (IT)**

(72) Inventors:
• **NUCCI, Cinzia**
**I-20132 Milano (IT)**
• **CORAZZA, Sabrina**
**I-20132 Milano (IT)**
• **LOHMER, Stefan**
**I-20132 Milano (IT)**
• **CAINARCA, Silvia**
**I - 20132 Milano (IT)**

(74) Representative: **Capasso, Olga et al**
**De Simone & Partners S.p.A.**
**Via Vincenzo Bellini, 20**
**00198 Roma (IT)**

(56) References cited:
**WO-A-00/28025          WO-A-01/18225**
**US-A- 5 714 666**

• **BLINKS J R: "USE OF PHOTOPROTEINS AS INTRACELLULAR CALCIUM INDICATORS" ENVIRONMENTAL HEALTH PERSPECTIVES, vol. 84, March 1990 (1990-03), pages 75-81, XP001205760 ISSN: 0091-6765**

## Description

The present invention relates to transgenic non human animals able to synthesize and express an apophotoprotein *in vivo* and to uses thereof.

## BACKGROUND OF THE INVENTION

Stem cells are unspecialized cells that are able to renew themselves through cell division for long periods (1). Moreover, under certain physiologic or experimental conditions, they can be differentiated into different cell types such as beating cardiomyocytes or insulin-producing cells of the pancreas (2, 3).

Stem cells can be subdivided and classified on the basis of their potency. Totipotent stem cells are produced from the fusion between an egg and a sperm cell. Cells produced by the first few divisions of the fertilized egg cell are also totipotent. These cells can grow into any type of cell. Pluripotent stem cells are the descendants of totipotent cells and can differentiate into any cell type except for totipotent stem cells. Multipotent stem cells can produce only cells of a closely related family of cells (e.g. blood cells such as red blood cells, white blood cells and platelets). Progenitor (sometimes called unipotent) cells can produce only one cell type, but have the property of self-renewal which distinguishes them from non-stem cells (3-6).

Stem cells can also be categorized according to their source, as either adult or embryonic. Adult stem cells are undifferentiated cells found among differentiated cells of a specific tissue and are mostly multipotent, capable of producing several but limited numbers of cell types. They comprise also newborn, umbilical cord, placental and amniotic fluid derived stem cells. They are also called somatic stem cells, or tissue stem cells, and are found in differentiated tissues in which, in a controlled manner, they differentiate and/or divide to produce all the specialized cell types of the tissue from which they originate (7-9).

Embryonic stem cells have the potential of becoming all types of specialized cells including germ cells (pluripotency). They have the capability of proliferating indefinitely in culture, under conditions that allow their proliferation without differentiation (3). Three types of pluripotent embryonic stem cells have been discovered up to now from rodents and humans (10):

- Embryonic Stem cells (ES) which derive from the inner cell mass of the pre-implantation blastocyst stage embryo. They have a normal karyotype. Several types of mouse and human embryonic stem cells are known and established, like' for example the mouse ES TBV2, R1, D3 cells (11-13, 63).
- Embryonic Carcinoma cells (EC) which derive from teratocarcinomas. Teratocarcinomas are gonadal tumors containing pluripotent stem cells present within a wide array of tissues derived from the three primary germ layers (endo-, meso-, and ecto-derm). The most used ones are the mouse EC P19 cell lines. These cells don't have a normal karyotype (14-18).
- Embryonic Germ cells (EG) which derive from cultured Primordial Germ Cells of the foetal genital ridge (PGCs). They have a normal karyotype but are abnormally genetically imprinted (19-22).

ES and EG cells can be injected into blastocysts of recipient mice giving rise to chimeric animals. In chimeric mice these pluripotent cells can contribute to every cell type, including the germline (20, 21, 23, 24). In contrast, murine EC cells introduced into embryos colonize most embryonic lineages, but generally do not colonize the gernaline, with one experimental exception (25-27). The inability of EC cells to form functional gametes most likely reflects their abnormal karyotype (28).

Stem cells are a very powerful tool for High Throughput Screening (HTS) technologies since they can be cultured and expanded *in vitro* for long periods, maintaining the self-renewal property, and they can undergo miniaturization. They allow the use of selectable and inducible markers for the preparation of a pure population ES cells. The technology of gene targeting/homologous recombination allows the Knock Out (KO) or Knock In (KI) of specific genes. Furthermore embryonic stem cells can differentiate into any cell type resembling primary cells (since they are non tumoral cells). In this way they offer a natural environment for the targets, they can address complex targets (like multi-subunit ion channels), that are regulated and expressed in a native way. This is a very important improvement since usually in HTS screening the cell-based assays are set up using tumoral cell lines and it is known that this tumoral environment can alter the physiological cell conditions. The use of pluripotent embryonic stem cells has acquired a fundamental role in the pharmaceutical field (29). For example for target evaluation, since understanding gene function in drug discovery is fundamental for success, murine ES cells represent a more rapid and less expensive tool compared to KO mice. In addition in case of a lethal KO, the use of ES cells could be very helpful for gene function evaluation.

Stem cells have also been used for the Embryonic Stem cell Test (EST) which was positively evaluated by the EVCAM study (European Centre for the Validation of Alternative Methods). This is a test of toxicology and teratology for drugs on the cellular and tissue differentiation generated from the 3 germ lineages (endo-, meso-and ecto-derm).

Stem cells are also very important for the analysis of the drugs secondary effects on the chronotropic activity on pulsing cardiomyocytes obtained by differentiation of pluripotent stem cells. This kind of test can reduce the number of animals used for toxicological studies. For example in the European Union up to 30000 chemicals that are currently on the market have to be re-evaluated in the next 10 years. This means the use of about 10 million animals. The creation of *in vitro* tests like the EST can be crucial in this sense and can also allow the testing of more chemicals in less time than conventional whole-animal experiments (30, 31, 32).

Bioluminescence is the phenomenon by which visible light is emitted by living organisms or by a substance derived from them through a variety of chemiluminescent reaction systems. Bioluminescence reactions require three major components: a luciferin (substrate), a luciferase (enzyme) and molecular oxygen. However, other components may also be required in some reactions, including cations ($Ca^{++}$ and $Mg^{++}$) and cofactors (ATP, NAD(P)H). Luciferases are enzymes that catalyse the oxidation of a substrate, luciferin, and produce an unstable intermediate. Light is emitted when the unstable intermediate decays to its ground state, generating oxyluciferin. There are many different unrelated types of luciferin, although many species from at least seven phyla use the same luciferin, known as coelenterazine. In some animals (e.g. jellyfish) the luciferin/luciferase system can be extracted in the form of a stable "photoprotein" which emits light upon calcium binding. Photoproteins differ from luciferases in that they are stabilized oxygenated intermediate complexes of luciferase and luciferin. Photoproteins are present in many marine coelenterates and allow these organisms to emit light for a variety of purposes including breeding, feeding and defense (33). There are many luminescent organisms, but only seven photoproteins, namely Thalassicolin (34,35), Aequorin (36,37,38), Mitrocomin (syn. with Halistaurin) (39,40), Clytin (syn. with Phialidin) (40, 41), Obelin (34,38,42,43), Mnemiopsin (44,45) and Berovin (44,45) have been isolated so far. All these proteins are complexes formed by an apoprotein, an imidazopyrazine chromophore (i.e., coelenterazine) and oxygen. Their amino acid sequences are highly conserved, especially in the region containing the three calcium binding sites (EF-hand structures). The term "photoprotein" identifies the coelenterazine-bound polypeptide, which is capable of luminescence, while "apophotoprotein" is used to indicate the protein without coelenterazine.

The most studied photoproteins are Aequorin, isolated from *Aequorea victoria* (46) and Obelin, isolated from *Obelia longissima* (47). The photoprotein may be regenerated from the apophotoprotein by incubation with coelenterazine, molecular oxygen, EDTA and 2-mercaptoethanol or dithiothreitol. Since coelenterazine is the common luminescent substrate used by the photoproteins Aequorin, Mitrocomin, Clytin and Obelin, the light-emitting reaction is likely the same in these four photoproteins (48,49,50,51).

The study of cellular events and their regulation requires sensitive, non invasive analytic methods. Photoproteins and in general the use of bioluminescence are excellent reporter systems as they have virtually no background in contrast to fluorescence systems.

Photoproteins are widely used in cell culture systems as reporter genes to monitor the cellular events associated with signal transduction and gene expression (33,34,46). Photoproteins are expressed in mammalian cells to monitor calcium changes in response to different stimuli. Intracellular calcium concentrations can be measured by adding the cofactor coelenterazine to mammalian cells expressing the apophotoprotein and detecting photon emission, which is indicative of intracellular calcium concentration. The use of cells which express both an apophotoprotein and a receptor involved in the modulation of intracellular calcium concentration provides a valid system for the screening of compounds for their effects on the release of intracellular calcium.

High throughput screening assays are often designed using a photoprotein as a reporter system. The sensitivity of the system as well as its high' signal to noise ratio allow the use of small assay-volumes. Calcium flux assays are commonly carried out in HTS format utilizing optical screening apparatus suited for the simultaneous analysis of a high number of samples and equipped with a luminescence imaging systems.

However, calcium concentration variation can also be detested using fluorescent calcium dyes like for example Fluo3, Fluo4, Fura2 and Calcium dyes (Molecular Devices and Molecular Probes) using fluorimetric instruments like FLIPR® (Fluorometric Imaging Plate Reader, Molecular Devices Corporation, Sunnyvale, CA, USA), one of the most used instruments in HTS assays. The apparatus is equipped with an optical detection device that allows signal isolation on a cell-monolayer, thereby enhancing sensitivity for cell-based assays.

The most recent FLIPR® system versions have been made suitable also for luminescence assays, even if with lower sensitivity compared to CCD camera-based equipments. To overcome the lower luminescence sensitivity of this system, photoproteins with enhanced light emission are highly advantageous.

The authors of the instant invention developed a non human transgenic animal obtained by genetic modification of the original genome, able to express an apophotoprotein. As animal model a transgenic mouse was obtained by injection of embryonic stem cells expressing an apophotoprotein into the blastocyst of a pseudopregnant female mouse, and crossing the resulting chimeras with wild type mice.

## DISCLOSURE OF THE INVENTION

It is an object of the invention a non human transgenic animal, the progeny and cell derivatives thereof able to express an apophotoprotein gene and produce a bioluminescent signal in response to intracellular calcium concentration variation.

A cell derivative is to be intended a cell, being a primary or a stem cell, a committed cell, a differentiated cell, a cell group, a tissue or an organ. The apophotoprotein gene expression and/or the production of a bioluminescent signal in response to intracellular calcium concentration variation is ubiquitous.

In a preferred aspect the apophotoprotein gene expression and/or the production of a bioluminescent signal in response to intracellular calcium concentration variation is inducible and/or organ-, tissue-, cell- or development stage-specific.

In a preferred embodiment the non human transgenic animal is a non human mammal, more preferably a rodent, most preferably a mouse. The non human transgenic animal of the invention is obtained by techniques known in the art, i.e. using embryonic stem cells able to express an apophotoprotein and produce a bioluminescent signal in the presence of a suitable chromophore substrate in response to intracellular calcium concentration variation. Photoproteins and in general bioluminescence are often used as effective reporter systems.

The non human transgenic animal of the invention is intended any non human animal obtained by genetic modification of the original genome, allowing the apophotoprotein expression. Typically, the transgenic animal is obtained with techniques known in the art, i.e. by injection of embryonic stem cells expressing a apophotoprotein into the blastocyst of a pseudopregnant female, and crossing the resulting chimeras with wild type animals.

Apophotoprotein is intended any apophotoprotein, natural or recombinant or synthetic. The apophotoprotein may be a natural or a mutagenized mutant, also having an improved luminescent activity and/or calcium sensitivity, or a chimeric protein derived from two different natural apophotoproteins, also further modified by deletion, addition or substitution of one or more amino acid residues, provided that the activity profile of the photoprotein, in terms of light-emission and calcium-responsiveness, is maintained or increased. Apophotoprotein sequences may also be optimized for mammalian codon usage and/or fused to mitochondrial target sequences (52,53,54). Apophotoproteins with enhanced bioluminescence are already disclosed in the prior art, i.e., the apophotoprotein Photina® (described in EP 1413584, and herein reported as SEQ ID No. 1), obtained by chimerization of the protein Obelin with a region of the Clytin protein.

Apophotoproteins with enhanced bioluminescence may also derive from mutagenesis as described in WO2006/094805, such as the Clytin sequence (GenBank accession number Q08121) mutagenised in the following position. $Gly_{142} \rightarrow Cys$; or the Clytin sequence (GenBank accession number Q08121) mutagenised in the following 12 positions: $Gly_{58} \rightarrow Glu$, $Asp_{69} \rightarrow Val$, $Ala_{70} \rightarrow Cys$, $Lys_{76} \rightarrow Arg$, $Lys_{71} \rightarrow Gly$, $Ile_{78} \rightarrow Cys$, $Asp_{81} \rightarrow Glu$, $Val_{86} \rightarrow Ile$, $Glu_{87} \rightarrow Ala$, $Ala_{90} \rightarrow Gln$, $Val_{92} \rightarrow Leu$, and $Glu_{97} \rightarrow Gln$.

The reporter apophotoprotein is cloned under the control of an ubiquitous promoter.

Stem cell is generally intended as a totipotent and/or pluripotent non human cell; or a human or non-human tumoral pluripotent cells, or a multipotent cell or a progenitor thereof, being of embryonic or of adult origin. Preferred stem cells for the generation of transgenic animals are totipotent and pluripotent non-human embryonic stem cells.

In particular preferred stem cells are mouse embryonic stem cells, preferably ES TBV2 (63) cells.

Typically, non-human embryonic stem cells are stably transfected with an expression vector containing an apophotoprotein encoding sequence.

The final positive clones expressing the apophotoprotein are chosen on the basis of their photoprotein content and on their ability to respond to adequate stimuli. non-human embryonic stem cells are injected into the blastocyst of a pseudopregnant female mouse. The chimeric mice generated in this way are then crossed with other mice in order to produce a transgenic heterozygous animal.

In order to obtain homozygous animals, two heterozygous animals are crossed together.

The non human transgenic animal of the invention is a valuable source of tissues or organs that can be used for pharmacological ex-vivo studies or for transplantation experiments. For example Langherans pancreatic islets can be used *ex-vivo* for monitoring calcium movements upon glucidic or depolarizing stimuli.

The non human transgenic animal of the invention is a valuable source of cell derivatives such as stem cells of adult and embryonic origin and/or primary cells containing apophotoprotein reporter genes, like for example macrophage precursors. In this context a cell derivative is a primary cell or a stem cell totipotent or pluripotent or multipotent or progenitor cell, being of embryonic or of adult origin.

Cell derivatives containing an apophotoprotein as reporter system can be used directly to generate cell based assays after loading with coelenterazine, or after injecting coelenterazine intravenously in the animal, and then adding the test molecule or stimulus.

Primary cell derivatives containing an apophotoprotein as reporter system can be used directly for screening purposes in order to identify agonists or antagonists for the specific target or as positive controls for the "primary-like" cells obtained after differentiation of the ES / photoprotein cells.

The stem cell derivative may be differentiated into a specific cell lineage to get the expression of a specific target. Stem cell derivatives can be used at the differentiated state to generate a cell based assay after loading with coelenterazine in order to identify agonists or antagonists for a specific target, or can be used at the undifferentiated state to identify pro or anti-differentiative agents.

Preferably the specific cell lineage is the muscle heart cell lineage or the neuronal lineage or those derived from hematopoietic or mesenchymal cell lineage.

The invention advantageously provides methods for the *in vivo* identification and/or testing of compounds for many applications, for example therapeutic, diagnostic applications.

In the context of the invention a compound library is a collection, either synthetic or recombinant, of compounds to be tested or identified.

It is another object of the invention a method for identifying a ligand able to stimulate a specific target so that a variation of intracellular $Ca^{++}$ is obtained comprising the steps of:

a) providing a cell derivative according to the invention, being primary or stem cells;
b) getting expression of said target, eventually also by means of differentiating said cells into a specific cell lineage;
c) loading cells with a suitable chromophore as substrate;
d) contacting cells with a compound library comprising putative ligands for said target;
e) detecting the photoprotein's bioluminescence.

Primary or stem cells may express the target endogeneously.

Preferably the specific cell lineage is the muscle heart cell lineage or the neuronal lineage or those derived from hematopoietic or mesenchymal cell lineage. More preferably the method is performed by High Throughput Screening. It is another object of the invention a method for identifying antagonists to a target, so that a variation of intracellular $Ca^{++}$ is obtained, comprising the steps of:

a) providing a cell derivative of a non human transgenic animal according to the invention, being primary or stem cells;
b) getting expression of said target, eventually also by means of differentiating said cells into a specific cell lineage;
c) loading cells with a suitable chromophore as substrate;
d) contacting cells with a compound library comprising putative antagonists for said target;
e) contacting cells with' a ligand able to stimulate the said target;
f) detecting the photoprotein's bioluminescence variation.

Primary or stem cells may express the target endogeneously.

Preferably the specific cell lineage is the muscle heart cell lineage or the neuronal lineage or those derived from hematopoietic or mesenchymal cell lineage.

More preferably the method is performed by High Throughput Screening.

Another object of the invention is a method for identifying an agent stimulating the differentiation of cell derivatives according to the invention towards a specific cell lineage comprising the steps of

a) providing a stem cell derivative of a non human transgenic animal according to claims 1 to 12 at an undifferentiated stage;
b) exposing said stem cell derivative to a compound library comprising putative inducing differentiation agents;
c) getting expression of at least one specific cell lineage target;
d) loading cells with a suitable chromophore as substrate;
d) stimulating said specific cell lineage target by a ligand so that a variation of intracellular $Ca^{++}$ is obtained;
e) detecting photoprotein's bioluminescence.

In a preferred embodiment the method is performed by High Throughput Screening.

Another object of the invention is a method for identifying an agent inhibiting the differentiation of stem cells derivatives according to the invention towards a specific cell lineage comprising the steps of

a) providing stem cell derivatives of a non human transgenic animal according to claims 1 to 12 at an undifferentiated stage;
b) exposing said stem cell derivatives to a compound library comprising putative inhibiting differentiation agents;
c) exposing said stem cell derivatives to a known inducing differentiation agent to get expression of at least one specific cell lineage target;
d) loading cells with a suitable chromophore as substrate;
e) stimulating said specific cell lineage target by a ligand so that a variation of intracellular $Ca^{++}$ is obtained;
f) detecting photoprotein's bioluminescence.

In a preferred embodiment the method is performed by high Throughput Screening.

It is another aspect of the invention the use of a cell derivative of the non human transgenic animal according to the invention as positive controls for the "primary-like" cells obtained after differentiation of ES / photoprotein cells.

It is another aspect of the invention the use of a cell derivative of the non human transgenic animal according to the invention wherein said cell derivative containing a photoprotein as reporter system is used for screening of compounds, typically agonists, antagonists, pro- or anti-differentiative agents. It is another aspect of the invention the use of tissue or organ derivatives of the non human transgenic animal according to the invention, containing a photoprotein as reporter system, for pharmacological ex-vivo studies and for transplantation experiments in animal models.

It is another aspect of the invention the use of the non human transgenic animal and the progeny thereof according to the invention for bio-imaging studies.

It is another aspect of the invention the use of the non human transgenic animal and the progeny thereof according to the invention for crossing with pharmacological relevant animal models. Preferably the animal model is a transgenic animal carrying reporter genes, or KO or KI mutant animal models. Such crosses allow the generation of new animal model progenies carrying the photoprotein reporter gene of the invention in combination with other transgenes (such as reporters) and/or mutated genes (such as those obtained by homologous recombinations).

It is another aspect of the invention the use of the non human transgenic animal according to the invention for *in vivo* testing of toxicity and/or teratology of a substance.

The invention will be described in more detail in the following experimental section by reference to the following figures:

**Fig. 1** Clone pool analysis of the 114 neomycin resistant clones. A. Histamine response kinetics (1 00 μM) was measured at CCD camera-based luminometer and recorded as RLU (Relative Luminescence Units) values. The experiments were performed in 96 MTP 24 h after cell **seeding.** CCD camera-based luminometer conditions: high sensitivity, for 60 seconds. **B.** Total photoprotein residual activity was measured after Triton. X-100 cell lysis. CCD camera-based luminometer conditions: low sensitivity, reading time 5 seconds.

**Fig. 2** Analysis of the 12 ES / mito c-Photina® best clones. **A.** Histamine response (100 μM) of the 12 best mito c-Photina® ES clones was measured in 96 MTP 24h after seeding 20000 cells/well. CCD camera-based luminometer conditions: high sensitivity, reading time 60 seconds. **B.** Total photoprotein residual activity of the 12 best mito c-Photina® ES clones was measured after Triton X-100 cell lysis. CCD camera-based luminometer conditions: low sensitivity, reading time 5 seconds.

**Fig. 3** Analysis of the 2 ES/ mito c-Photina® final clones. **A.** Histamine response (100 μM) was measured in 96 MTP 24 h after seeding 10000 cells/well. CCD camera-based luminometer conditions: high sensitivity, reading time 60 seconds. **B.** Histamine response (100 μM) was measured in 96 MTP 24 h after seeding 20000 cells/well. CCD camera-based luminometer conditions: high sensitivity, reading time 60 seconds.

**Fig. 4** Immunofluorescence analysis on undifferentiated ES / mito c-Photina® / 29 clone. Immunofluorescence assay performed using the following antibodies:

> **A.** Mouse anti-SSEA-1 primary antibody + anti-mouse FITC secondary antibody.
> **B.** Mouse anti-oct3/4 primary antibody + anti-mouse FITC secondary antibody.

**Fig. 5** Alkaline phosphatase activity measured with the ELF® Phosphatase staining kit.

**Fig. 6** *In vitro* cardiomyocytic differentiation assay on ES / mito c-Photina® / 29 clone.

> A. Embryoid Bodies in suspension at differentiation day 2.
> B. Embryoid Bodies in suspension at differentiation day 5 before the plating on gelatin-coated dishes.
> C. Percentage of Embryoid Bodies containing pulsing areas evaluated by morphological analysis.

**Fig. 7** Immunofluorescence analysis on *in vitro* differentiated cardiomyocytes from ES / mito c-Photina® / 29 clone. Immunofluorescence assay was performed using the following antibodies:

> **A.** Mouse anti-sarcomeric alpha-actinin primary antibody + anti-mouse FITC secondary antibody.
> **B.** Rabbit anti-Myosin Heavy Chain (MHC) primary antibody + anti-rabbit FITC secondary antibody.
> **C.** Rabbit anti-GATA4 primary antibody + anti-rabbit FITC secondary antibody.

**Fig. 8** CCD camera-based luminescent functional test on *in vitro* differentiated cardiomyocytes from ES / mito c-Photina® / 29 clone.

> **A.** Tyrode buffer, Norepinephrine (100 μM) and Endothelin-1 (50 nM) responses were measured in 96 MTP, 4 h after seeding 20000 cells/well. CCD camera-based luminometer conditions: high sensitivity, reading time 60 seconds.

**B.** Depolarization stimulus (40 mM. KCl) measured in presence or in absence of 5 μM Nifedipine. CCD camera-based luminometer conditions: high sensitivity, reading time 60 seconds.

**C.** Total photoprotein residual activity was measured after Triton X-100 cell lysis. CCD camera-based luminometer conditions: high sensitivity, reading time 30 seconds.

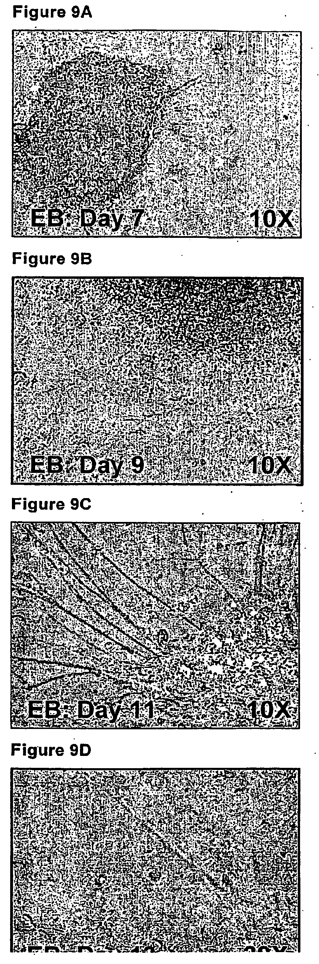

**Fig. 9** Visible imaging of *in vitro* neuronal differentiation assay on ES / mito c-Photina® / 29 clone.

**A. Embryoid** Bodies plated on gelatin-coated dishes at differentiation day 7.
**B.** Embryoid Bodies plated on gelatin-coated dishes at differentiation day 9.
**C.** Embryoid Bodies plated on gelatin-coated dishes at differentiation day 11.
**D.** Embryoid Bodies plated on gelatin coated dishes at differentiation day 13.

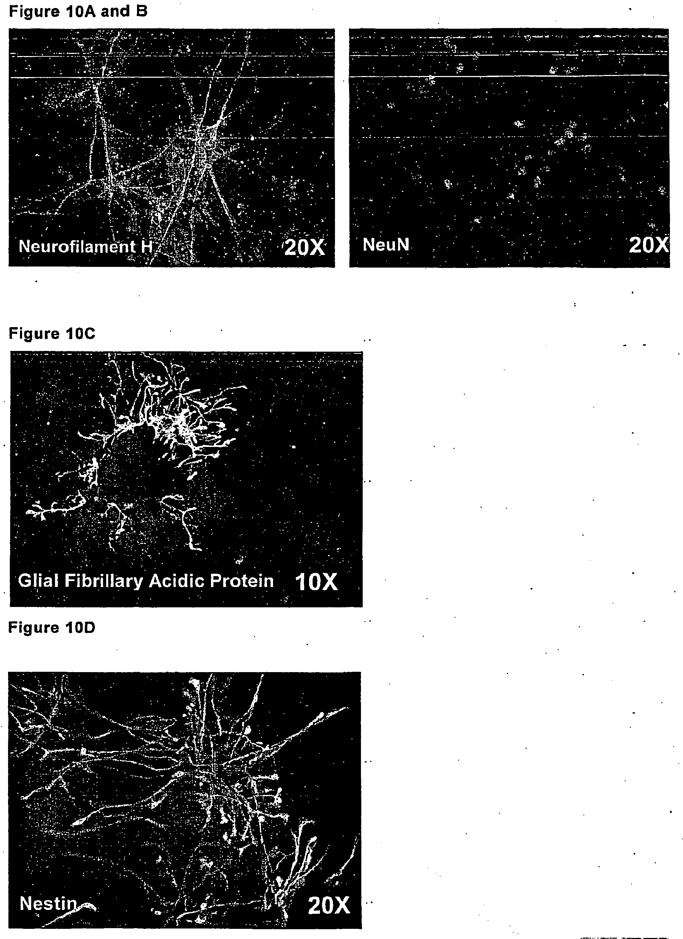

**Fig. 10** Immunofluorescence analysis on *in vitro* differentiated neurons from ES / mito c-Photina® / 29 clone on 96 MTP. Immunoflurescence assay was performed using the following primary antibodies:

**A.** Rabbit anti-Neurofilament H (NF H) primary antibody + anti-rabbit FITC secondary antibody (same field as **B.**).
**B.** Mouse anti-Neuronal Nuclei (NeuN) primary antibody + anti-mouse rhodaminated secondary antibody (same field as **A.**).
**C.** Rabbit anti-Glial Fibrillary Acidic Protein (GFAP) primary antibody + anti-rabbit FITC secondary antibody.
**D.** Mouse anti-Nestin primary antibody + anti-mouse FITC secondary antibody.

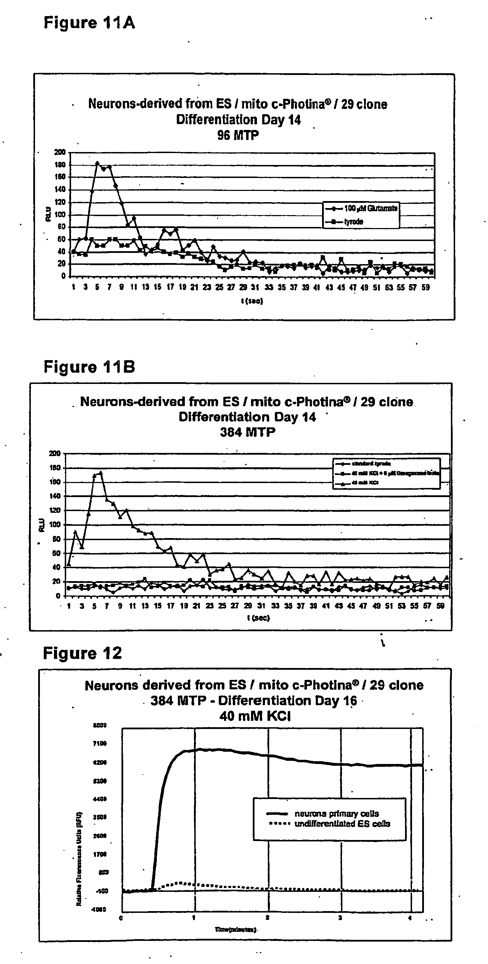

**Fig. 11** CCD camera-based luminescent functional test on *in vitro* differentiated neurons from ES / mito c-Photina® / 29 clone.

**A.** Standard tyrode, and Glutamate (100 μM) responses were measured in 96 MTP on day 14. CCD camera-based luminometer conditions: low sensitivity, reading time 60 seconds.
**B** Standard tyrode, and 40 mM KCl responses were measured in presence or in absence of 6 μM Omegaconotoxin GVIA in 384 MTP on day 14. CCD camera-based luminometer conditions: high sensitivity, reading time 60 seconds.

**Fig. 12** FLIPR$^{384}$ functional test on *in vitro* undifferentiated and differentiated (at day 16) neurons from ES / mito c-Photina® / 29 clone. For FLIPR$^{384}$ analysis the cells were incubated for 30 min at 37˚C with Membrane Potential Assay kit and then injected.with. KCl at 40 mM final concentration. Fluorescence signal was recorded for 250 sec and expressed as RFU (FLIPR$^{384}$ settings: Exp. Time: 0.3 sec; injection speed: 20 μl/sec; injection height: 50 μl; reading time: 360 seconds).

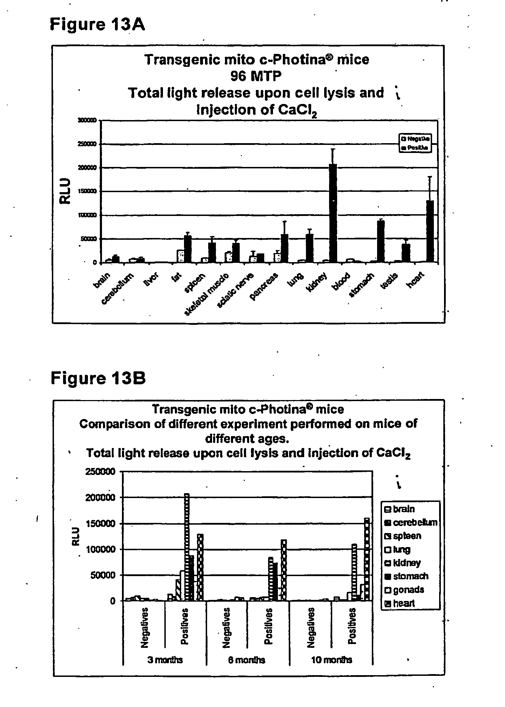

**Fig. 13** Photoprotein tissue localisation in transgenic mito c-Photina® mice. **A.** 14 different tissues/organs were explanted from a negative and a positive mouse expressing mito c-Photina®, and incubated for 3 h with 20 μM coelenterazine at room temperature. The photoprotein content in each tissue was measured injecting a solution of 250 mM CaCl$_2$ and Triton X-100. CCD camera-based luminometer conditions: high sensitivity, reading time 60 seconds. **B.** Comparison of 8 tissue/organs explanted from negative and positive mice of different ages (3, 6, 10 months old) expressing mito c-Photina®, and incubated for 3 h with 20 μM coelenterazine at room temperature. The photoprotein content in each tissue was measured injecting a solution of 250 mM CaCl$_2$ and Triton X-100. CCD camera-based luminometer conditions: high sensitivity, reading time 60 seconds.

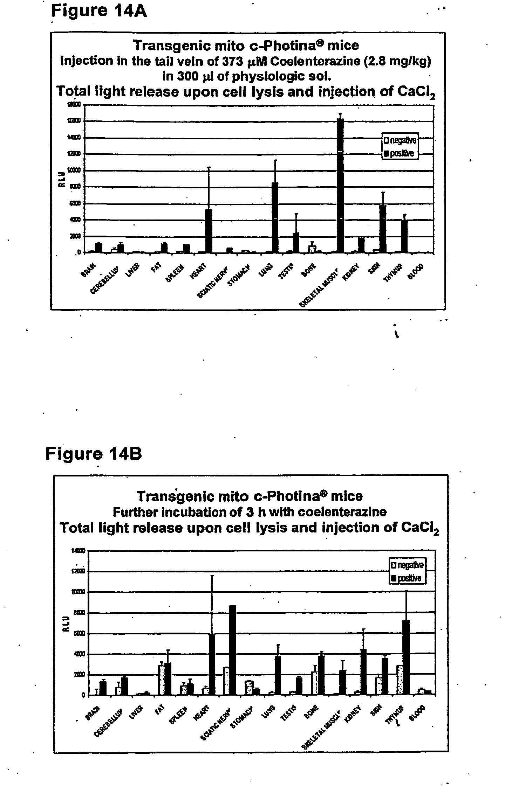

**Fig. 14** Tissues and organs incubated with coelenterazine in different conditions. **A.** CCD camera-based luminometer analysis of different tissues/organs after intra-systemical injection of 2.8 mg/kg coelenterazine in a positive transgenic mito c-Photina® mouse and in a negative control. After 3 h, 16 different tissues/organs were explanted from both mice. Half of the material was directly seeded in a white 96 MTP. **B.** CCD camera-based luminometer analysis of the remaining material further incubated at room temperature for 3 h with 20 μM coelenterazine. The photoprotein content of all the samples was analysed at CCD camera based luminometer by recording the light emitted after injection of 250 mM CaCl$_2$ and Triton X-100 solution. CCD camera-based luminometer conditions: High sensitivity, reading time 60 seconds.

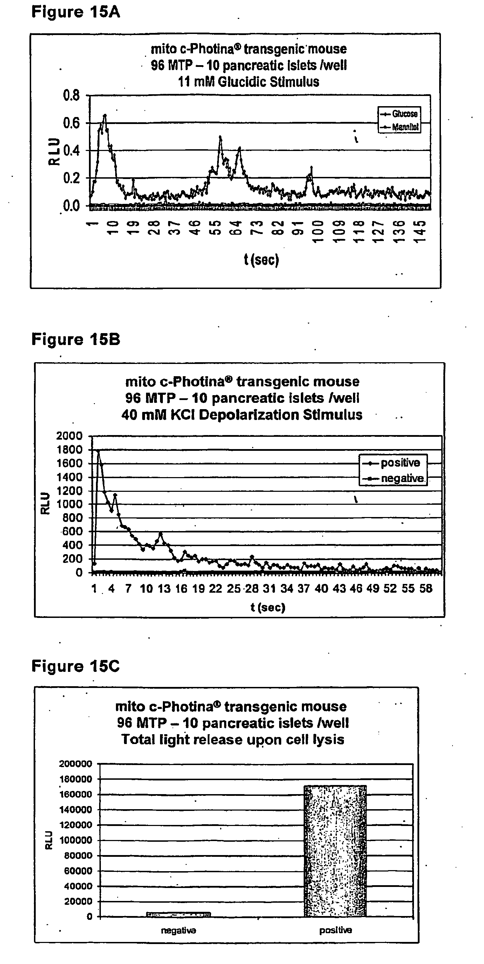

**Fig. 15** CCD camera-based luminometer functional test performed on pancreatic islets isolated from transgenic mito c-Photina® mice. Pancreatic islets were isolated from 1 positive transgenic mito c-Photina® and 1 negative mouse. After an overnight culture, 10 islets/well were put in a white 96 MTP.

**A.** Two different wells containing 10 islets each were incubated for 3 h with 10 μM coelenterazine and stimulated respectively with 11 mM Glucose or 11 mM Mannitol as negative control. Luminoskan luminometer. Integration time 0.5 sec. Reading time 150 seconds.

**B.** The islets were then stimulated with 40 mM KCl. Light emitted was measured at a CCD camera-based luminometer with the following settings: high sensitivity, reading time 60 seconds.

**C.** Islets total photoprotein residual activity was evaluated recording the light emitted after cell lysis with a solution containing Triton X-100. CCD camera-based luminometer conditions: high sensitivity, reading time 60 seconds.

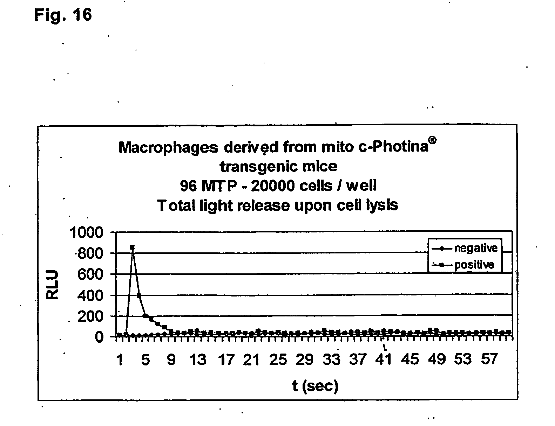

**Fig. 16** Total light release measured upon cell lysis of c-Photina® transgenic mouse-derived macrophages. 20000 cells/well were seeded in a 96 MTP plate. CCD camera-based luminometer conditions: high sensitivity, integration time 0.6 sec, reading time 60 seconds.

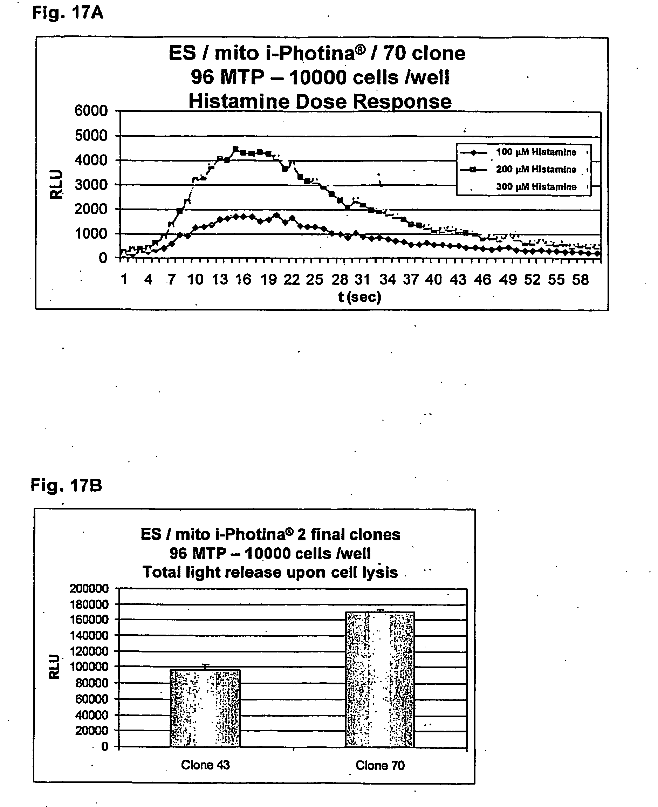

**Fig. 17** Analysis of the 2 ES / mito i-Photina® final clones. A. Histamine dose response of clone N. 70 measured in 96 MTP 24 h after seeding 10000 cells/well. CCD camera-based luminometer conditions: high sensitivity, reading time 60 seconds. B. Total photoprotein residual activity of the 2 best ES / mito i-Photina® clones was measured after cell lysis with a solution containing Triton X-100. CCD camera-based luminometer conditions: low sensitivity, reading time 5 seconds.

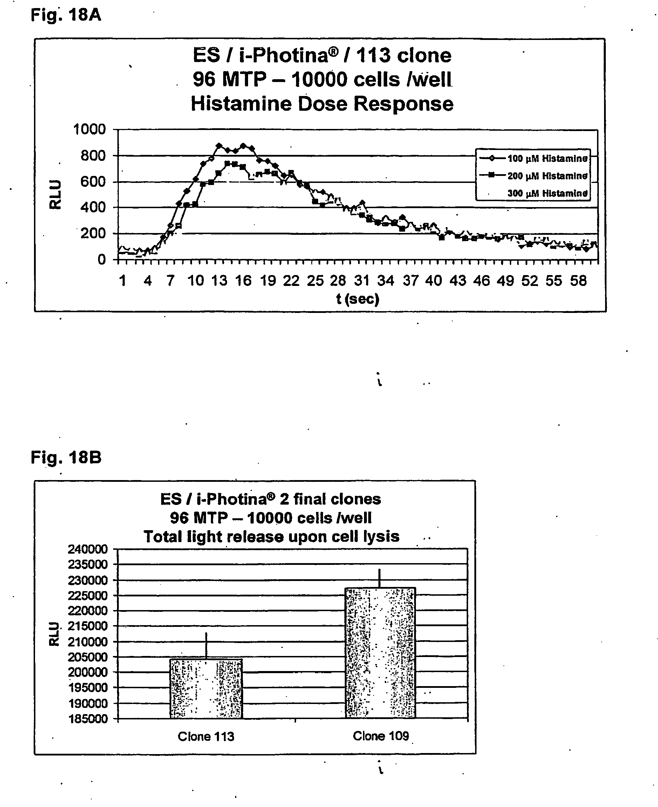

**Fig. 18** Analysis of the 2 ES / i-Photina® final clones. **A.** Histamine dose response of clone N. 113 measured in 96 MTP 24 h after seeding 10000 cells/well. CCD camera-based luminometer conditions: high sensitivity, reading time 60 seconds. **B.** Total photoprotein residual activity of the 2 best ES / i-Photina® clones was measured after cell lysis with a solution containing Triton X-100. CCD camera-based luminometer conditions: low sensitivity, reading time 5 seconds.

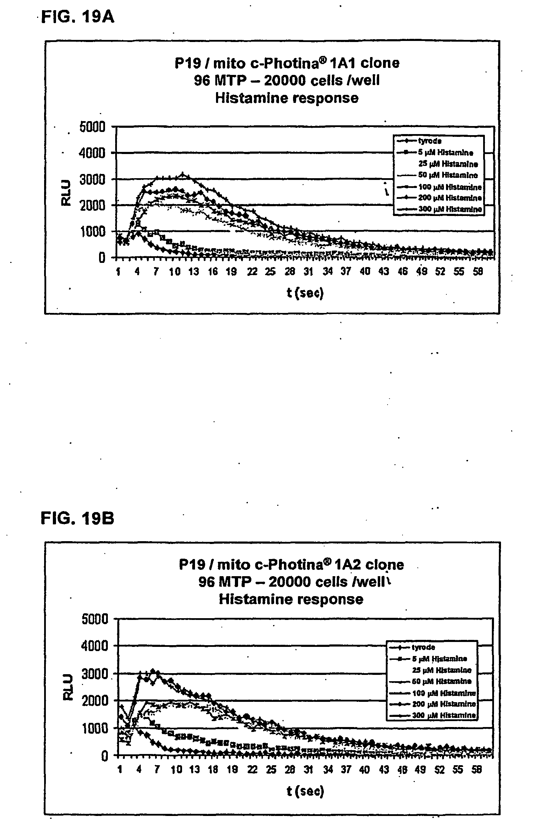

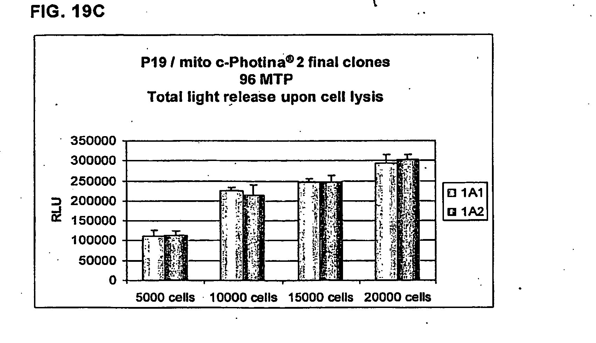

**Fig. 19** Analysis of the P 19 / mito c-Photina® 2 final clones. A. Histamine response of the P19 / mito c-Photina® 1A1 clone was measured in 96 MTP 24 h after seeding 20000 cells/well. CCD camera-based luminometer conditions: high sensitivity, reading time 60 seconds. **B.** Histamine response of the P19 / mito c-Photina® 1A2 clone was measured in 96 MTP 24 h after seeding 20000 cells/well. CCD camera-based luminometer conditions: high sensitivity, reading time 60 seconds. C. Total photoprotein residual activity was measured after Triton X-100 cell lysis. CCD camera-based luminometer conditions: low sensitivity, reading time 30 seconds.

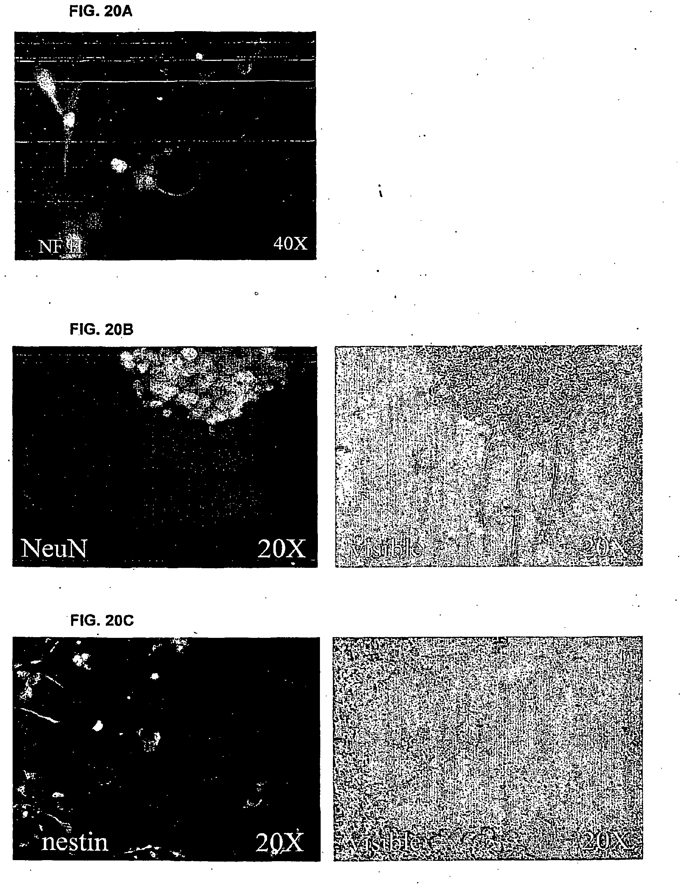

**Fig. 20** Immunofluorescence analysis on *in vitro* differentiated neurons from P19 / mito c-Photina® / 1A1 clone on 96 MTP. Immunofluorescence assay was performed using the following primary antibodies:

**A.** Rabbit anti-NeuroFilament H (NF H) primary antibody + anti-rabbit FITC secondary antibody.
**B.** Mouse anti-Neuronal Nuclei primary antibody + anti-mouse rhodaminated secondary antibody as seen with fluorescent and visible light microscopy.
C. Mouse anti-Nestin primary antibody + anti-mouse FITC secondary antibody as seen with fluorescent and visible light microscopy.

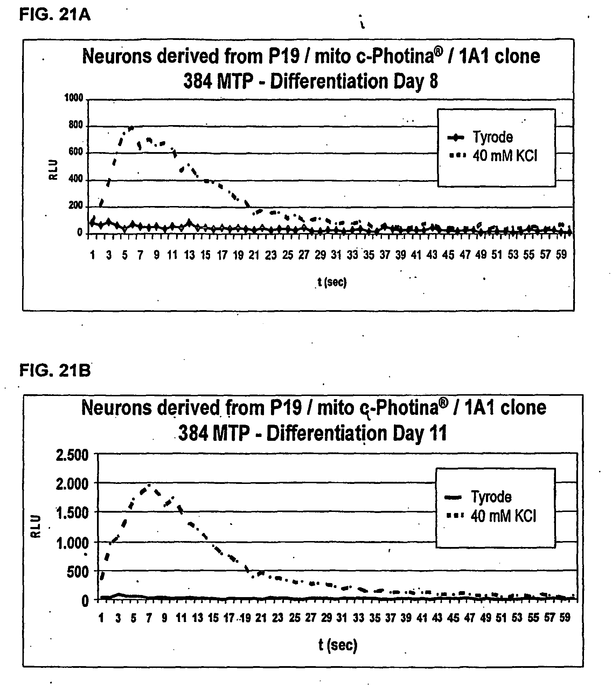

**Fig. 21** CCD camera-based functional test on *in vitro* differentiated neurons from P19 / mito c-Photina® / 1A1 clone.

**A.** Tyrode, and 40 mM KCl responses were measured in 384 MTP on day 8 differentiated neurons. CCD camera-based luminometer conditions: high sensitivity, reading time 60 seconds. **B.** Tyrode, 40 mM KCl responses were measured in 384 MTP on day 11 differentiated neurons. CCD camera-based luminometer conditions: high sensitivity, reading time 60 seconds.

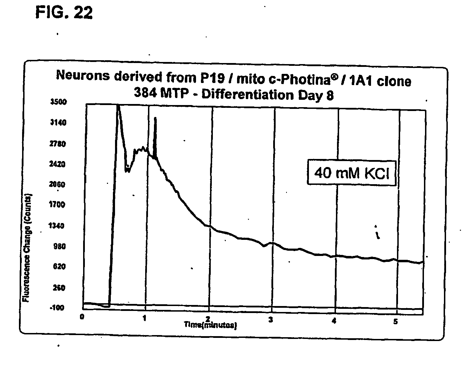

**Fig. 22** FLIPR[384] functional test on *in vitro* differentiated neurons from P 19 / mito c-Photina® / 1A1 clone at day 8. For FLIPR[384] analysis the medium was replaced with Fluo-4 NW® calcium sensitive fluorescent dye. The plate was then incubated for 30 min at 37˚C and 30 min at room temperature and then injected with KCl solution (40 mM final concentration). The fluorescence signal was recorded for 360 sec and expressed as RFU (FLIPR[384] settings: Exp. Time: 0.3 sec; injection speed: 20 $\mu$l/sec; injection height: 50 $\mu$l; reading time: 360 seconds).

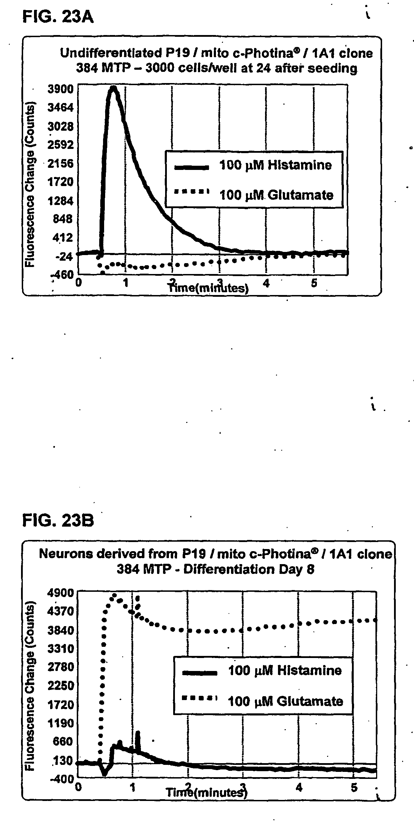

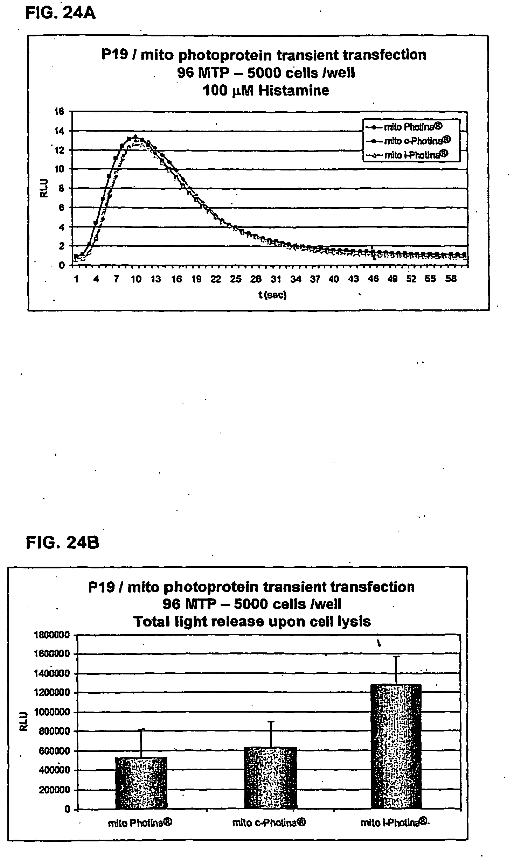

**Fig. 23** FLIPR[384] functional test on undifferentiated and *in vitro* differentiated neurons at day 8 from P19 / mito c-Photina® / 1A1 clone. For FLIPR[384] analysis the medium was replaced with 25 $\mu$l/well of Flu-4 NW® calcium sensitive fluorescent dye. Plates were then incubated for 1 h and then injected with Histamine or Glutamate (100 $\mu$M final concentration). The fluorescence signal was recorded for 360 sec and expressed as RFU (FLIPR[384] settings: Exp. Time: 0.3 sec; injection speed: 20 $\mu$l/sec; injection height: 50 $\mu$l; reading time: 330 seconds). A. FLIPR[384] responses of undifferentiated P19 / mito c-Photina® / 1A1 clone cells 3000 cells/well tested 24 hrs after seeding. B. FLIPR[384] responses of differentiated neurons from P19 / mito c-Photina® / 1A1 clone at day 8. **Fig. 24** Transient transfection of different photoproteins (mito Photina®, mito c-Photina®, mito i-Photina®) in P 19 cells. **A.** 100 $\mu$M Histamine response was measured at Luminoskan luminometer. Reading time 60 seconds. **B.** Total light emission was measured after cell lysis with Triton X-100 at Berthold luminometer. Reading time 3 seconds.

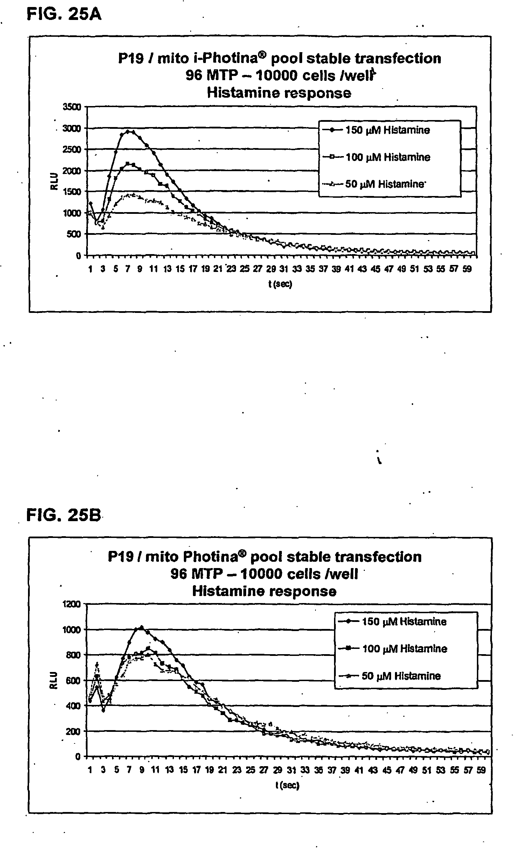

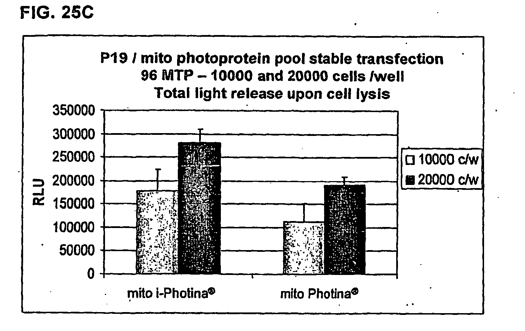

**Fig. 25** Analysis of the pool of mito i-Photina® **(A.),** and mito Photina® **(B.)** stably transfected P19 cells. 50, 100

and 150 μM Histamine dose-response in 96 MTP was measured at 24 h after cell seeding of 10000 cells/well. CCD camera-based luminometer conditions: high sensitivity, reading time 60 seconds. C. Total light emission was measured after cell lysis with Triton X-100 in 96 MTP 24 h after seeding 10000 and 20000 cells/well. CCD camera-based luminometer conditions: low sensitivity, reading time 30 seconds.

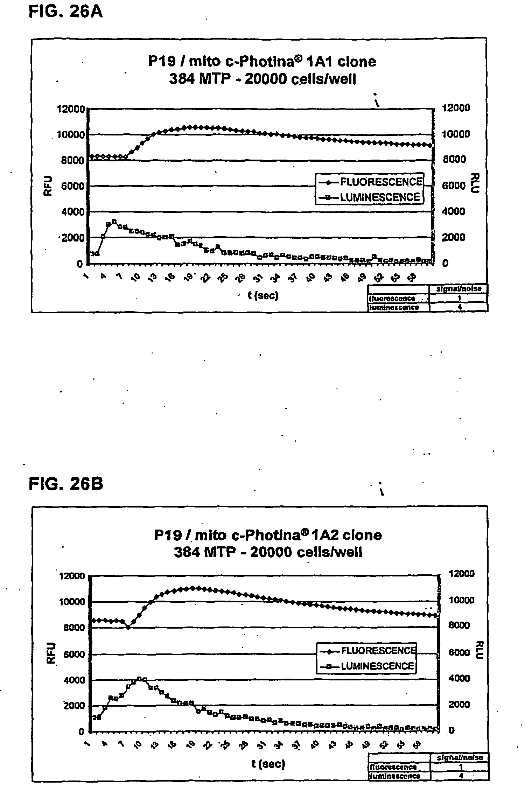

**Fig. 26** Comparison at FLIPR and CCD camera-based luminometer of the P19 mito c-Photina® 2 final clones. Histamine response of the P19 / mito c-Photina® 1A1 clone **(A.)** and of the P19 / mito c-Photina® 1A2 clone **(B.)** was measured in 384 MTP 24 h after seeding 20000 cells/well. For CCD camera-based luminometer analysis medium was replaced 4 h before reading with 10 μM coelenterazine solution at 37°C (CCD camera-based luminometer conditions: high sensitivity, reading time 60 seconds). For FLIPR® analysis the medium was replaced 30 min before reading with Calcium 3 Assay kit 0.5X (FLIPR384 settings: Exp. Time: 0.2 sec; injection speed: 20 μl/sec; injection height: 50 μl; reading time: 60 seconds). For both measurements the final concentration of Histamine was 100 μM. The signal to noise ratio was calculated and reported both for luminometer (expressed in RLU: Relative Luminescence Units) and fluorometer (expressed in RFU: Relative Fluorescence Units).

**MATERIALS AND METHODS**

**Photoprotein description**

**Photina®**

The chimeric photoprotein Photina® is described in Patent EP 1413584, herein reported as SEQ ID No. 1:

```
Met Ser Ser Lys Tyr Ala Val Lys Leu Lys Thr Asp Phe Asp
Asn Pro Arg Trp Ile Lys Arg His Lys His Met Phe Asp Phe
Leu Asp Ile Asn Gly Asn Gly Lys Ile Thr Leu Asp Glu Ile
Val Ser Lys Ala Ser Asp Asp Ile Cys Ala Lys Leu Gly Ala
Thr Pro Glu Gln Thr Lys Arg His Gln Asp Ala Val Glu Ala
Phe Phe Lys Lys Ile Gly Met Asp Tyr Gly Lys Glu Val Glu
Phe Pro Ala Phe Val Asp Gly Trp Lys Glu Leu Ala Thr Ser
Glu Leu Lys Lys Trp Ala Arg Asn Glu Pro Thr Leu Ile Arg
Glu Trp Gly Asp Ala Val Phe Asp Ile Phe Asp Lys Asp Gly
Ser Gly Thr Ile Thr Leu Asp Glu Trp Lys Ala Tyr Gly Lys
Ile Ser Gly Ile Ser Pro Ser Gln Glu Asp Cys Glu Ala Thr
Phe Arg His Cys Asp Leu Asp Asn Ser Gly Asp Leu Asp Val
Asp Glu Met Thr Arg Gln His Leu Gly Phe Trp Tyr Thr Leu
Asp Pro Glu Ala Asp Gly Leu Tyr Gly Asn Gly Val Pro
```

**i-Photina®**

i-Photina® (Patent Application EP05005390.9) is obtained by mutagenesis Gly$_{142}$→Cys of the Clytin photoprotein (GenBank accession number Q08121).

**c-Photina®**

The c-Photina® (Patent Application EP06000171) is obtained mutating the Clytin sequence (GenBank accession number Q08121) in the following 12 positions:

Gly$_{58}$→Glu, Asp$_{69}$→Val, Ala$_{70}$→Cys, Lys$_{76}$→Arg, Lys$_{77}$→Gly, Ile$_{78}$→Cys, Asp$_{81}$→Glu, Val$_{86}$→Ile, Glu$_{87}$→Ala,

Ala$_{90}$→Gln, Val$_{92}$→Leu, and Glu$_{97}$→Gln.

**Photoprotein optimization for expression in mammalian cells**

The codon usage of the *c-Photina*® and *i-Photina*® genes were adapted to the codon bias of highly expressed mammalian genes. In addition regions of very high (>80%) or very low (<30%) GC content have been avoided where possible.
For efficient translation initiation the Kozak-consensus sequence was introduced upstream of the start codon. Two STOP codons were added to ensure efficient termination.

**Cloning procedure**

The genes were cloned in the pcDNA3.1+ vector (Invitrogen) with or without the mitochondrial tag (mito) to obtain pcDNA3 mito c-Photina®, pcDNA3 mito i-Photina®, and pcDNA3 i-Photina®. For the mitochondrial targeting (52-54) the human Cytocrome c oxydase, subunit VIII, signal sequence was used:

5'-ATGTCCGTCCTGACGCCGCTGCTGCTGCGGGGCTT
GACAGGCTCGGCCCGGCGGCTCCCAGTGCCGCGCGC
CAAGATCCATTCGTTGGGATCCGCCACC-3' (SEQ ID No. 2).

The construct obtained was verified by full-length dideoxy sequencing.

**ES Cell Culture**

ES cells were cultured using standard methods (55, 56).

ES Culture medium, seeding and incubation :

TBV2 (12952/SvPas) Embryonic stem cells (63) are cultured with 15% Foetal Bovine Serum, FBS (ES qualified, Invitrogen, Cat. N. 16141079) DMEM Dulbecco's Modified Eagles Medium, high glucose, without NaPiruvate (Invitrogen, Cat. N. 10313021), 100 $\mu$M $\beta$-Mercaptoethanol (Invitrogen, Cat. N. 31350010), 2 mM Glutamine (Invitrogen, Cat. N. 25030024), 1000 U/ml Leukemia Inhibitory Factor, LIF (Prodotti Gianni, Cat. N. ESG1107) at 37˚C, 5% CO$_2$. Primary Mouse Embryonic Fibroblasts (MEF) cells are cultured 10% Foetal Bovine Serum, FBS (Celbio, Cat. N. CHA11152) DMEM Dulbecco's Modified Eagles Medium, high glucose (Invitrogen, Cat. N. 10313021), 1 mM Sodium Pyruvate (Invitrogen, Cat. N.11360039) non essential aminoacids (Invitrogen, Cat. N. 11140-035), 2 mM Glutamine (Invitrogen, Cat. N. 25030024) at 37˚C, 5% CO$_2$.

Stable transfection

DNA constructs corresponding to the photoproteins were transfected using electroporation methods. 30-40 $\mu$g of the *mito c-Photina*® *mito i-Photina*® and *i-Photina*® DNA, linearized with BglII (New England Biolabs), were transfected using 7x10$^6$ ES cells (electroporation condition: 500 $\mu$F, 0.24 kV, BioRad gene pulser) and incubate on ice for 10-20 minutes. The cell suspension was diluted in ES cell medium containing LIF and transferred on gelatinized 100 mm-diameter plates. After approximately 48 hours selection was started using ES media containing 200 $\mu$g/ml G148 (Geneticin, SIGMA, Cat. N. G5013).
Colonies were generally ready for picking 8-9 days after electroporation.

Clone Selection Process:

1. 114 ES pcDNA3/ mito c-Photina®, 130 ES pcDNA3/ mito i-Photina® and 99 ES pcDNA3/ i-Photina® clones were picked.
2. 24 h and 48 h after seeding, the transfected cells were plated in 2x96MTP white plates in ES medium with LIF.
3. Medium was replaced with 50 $\mu$l/well of tyrode (130 mM NaCl, 5 mM KCl, 2 mM CaCl$_2$, 1 mM MgCl$_2$, 5 mM

NaHCO$_3$ and 20 mM HEPES, pH 7.4, 2 mM Ca$^{2+}$) and coelenterazine 10 µM (Pharma Tech International).
4. Positive clones were selected evaluating:

- Response to 100 µM Histamine (Sigma, Cat. N. H7125-5G). CCD camera-based luminometer conditions: high sensitivity, reading time 60 seconds.
- Residual total photoprotein activity measured after Triton X-100 cell lysis. CCD camera-based luminometer conditions: low sensitivity, reading time 5 seconds. 12 clones for each construct were chosen and expanded and retested at counted cells 10000-20000 c/w in 96 MTP and 2500 c/w in 384 MTP.
- Response to 100 µM Histamine. CCD camera-based luminometer conditions: high sensitivity, for 60 seconds.
- Residual total photoprotein activity measured after Triton X-100 lysis. CCD camera-based luminometer conditions: low sensitivity, for 5 seconds.

**DNA extraction**

DNA from ES cells plated on gelatin coated dishes was extracted with standard Proteinase K digestion and phenol-clorophorm-isopropanol extraction method (59).

**Determination of the number of insertions by Quantitative PCR**

QPCR (Quantitative Polymerase Chain Reaction) was performed on ES /mito c-Photina® cells using approximately 3 ng of DNA per reaction with the "Platinum® SYBR Green® QPCR SuperMix UDG" protocol (60, Invitrogen). The primers used were designed using the Primer Express® Software v2.0 (Applied Biosystems), on c-Photina® (CPH) and *neomycin* (neo) genes to detect the plasmid used in the transfections, and specific to the *gusB* gene to detect the genomic DNA:

CPH-for: CACCAAGTGTGCGTGGAGG (SEQ ID No. 3);
CPH-rev: GCGATCTCCTTGCCGTACTC (SEQ ID No. 4);
neo-for: CACGTACTCGGATGGAAGCC (SEQ ID No. 5);
neo-rev: CCCTGATGCTCTTCGTCCAG (SEQ ID No. 6);
gusB-for: GGAGGTGATTCAGCCACAGC (SEQ ID No. 7);
gusB-rev: TCGGCTTCTGATGCGTCTTA (SEQ ID No. 8).
All QPCR experiments were run on an ABI Prism 7700 Sequence Detector (Applied Biosystems).

The PCR protocol was the following: 50˚C for 2 min hold, 95˚C for 2 min hold, 40 cycles of: 95˚C, 15 sec, 60˚C, 1 min; 95˚C for 15 sec. 20 min-long temperature gradient from 60˚C to 95˚C (melting curve step).
At the end of the run, fluorescence data acquired during PCR were processed as described in the ABI Prism 7700 user's manual.
The melting temperature profile analysis of the PCR products was made using the "Dissociation Curves 1.0" software (Applied Biosystems). No primer-dimers were produced in any of the QPCR experiments.
To calculate the number of copies of *neomycin* and/or *c-Photina*® gene per diploid genome (i.e., per cell) we entered the Cts (Cycle Threshold) and the PCR efficiencies in the following formula:

$$\text{\# of copies per cell} = 2 \times \frac{(\text{PCR Efficiency }_{target})^{-(Ct\ target)}}{(\text{PCR Efficiency }_{gusB})^{-(Ct\ gusB)}}$$

where:

PCR Efficiency $_{target}$ = PCR efficiency of the *neomycin* or the *c-Photina*® gene;
PCR Efficiency $_{gusB}$ = PCR efficiency of the *gusB* gene; .
Ct *target* = Ct of the *neomycin* or the *c-Photina*® gene;
Ct *gusB* = Ct of the *gusB* gene.

The fraction on the right of the formula gives the number of copies of insert DNA per *gusB* copy. Since two *gusB* copies are present in a diploid genome, the fraction is multiplied by two.

**Southern Blot**

10 μg of ES genomic DNA of ES /mito c-Photina® cells was digested with different restriction enzymes, HindIII, XbaI, BamHI, HindIII/XbaI (Biolabs), loaded on 0.8% agarose gel, and transferred on a nylon membrane positively charged (Roche, Cat. N. 1417240). As probe was used the [$^{32}$P]dCTP -labelled c-*Photina*® coding sequence (59).

10 μg of ES genomic DNA of ES / mito i-Photina® and of ES / i-Photina® cells was digested with EcoRI restriction enzyme (Biolabs), loaded on 0.8% agarose gel, and transferred on a nylon membrane positively charged (Roche, Cat. N. 1417240). As probe was used the [$^{32}$P]dCTP -labelled *i-Photina*® coding sequence (59).

**Immunofluorescence Analysis**

1. The medium was removed and 3 washes with 1X PBS were performed.
2. The ES cells were fixed with 4% Paraformaldeide (PFA, MERCK, Whitehouse Station, NJ, USA, Cat. N. 1.04005. 1.000) solution for 20 min at room temperature.
3. The fixing solution was removed, and 3 washes with 1X PBS were performed at room temperature.
4 The blocking and permeabilization procedure was performed incubating the cells with 10% Normal Goat Serum (Cheinicon, Cat. N. S26-100ml) / 0.2% Triton X=100 in 1X PBS for 30 min at room temperature.
5. The blocking solution was removed, and 2 washes with 1X PBS were performed at room temperature.
6. The different antibodies were incubated in 10% Normal Goat Serum 0.1% Triton X-100 in 1X PBS for 2h at room temperature.

- The mouse monoclonal antibody anti oct 3/4 (C-10) (Santa Cruz Biotechnology, Cat. N. SC-5279) was used at a 1:100 dilution.
- The mouse monoclonal antibody anti SSEA-1 (Santa Cruz Biotechnology, Cat. N. SC21702) was used at a 1: 100 dilution in a buffer that did not contain the Triton X-100 permeabilizing agent.
- The rabbit polyclonal antibody anti Myosin Heavy Chain (MHC) (H-300) (Santa Cruz Biotechnology, Cat. N. SC-20641) was used at a 1:50 dilution.
- The rabbit polyclonal antibody anti GATA-4 (H-112) (Santa Cruz Biotechnology, Cat. N. SC-9053) was used at a 1:50 dilution.
- The mouse monoclonal antibody anti sarcomeric alpha-actinin (EA-53) (SIGMA, Cat. N. A 7811) was used at a 1:50 dilution.
- The rabbit polyclonal antibody anti NeuroFilament H (NF-H) (Chemicon, Cat. N. AB1989) was used at a 1: 100 dilution.
- The mouse monoclonal antibody anti Neuronal Nuclei (NeuN) (Chemicon, Cat. N. MAB377) was used at a 1: 100 dilution.
- Thee rabbit polyclonal antibody anti Glial Fibrillary Acidic Protein (Dako, Cat. N. Z0334) was used at a 1:100 dilution.
- The mouse monoclonal antibody anti Nestin (Rat-401) (Chemicon, Cat. N. MAB353) was used at a 1:100 dilution.

7. 3 washes with 1X PBS were performed at room temperature.
8. The incubation with the secondary antibody was performed with a fluoresceinated anti-mouse, or anti rabbit FITC secondary antibody alone or in combination with a rhodaminated anti-mouse antibody (Goat and Mouse IgG/IgM Rhodamine. Chemicon, Cat. N.AP130R) in 10% Normal Goat Serum/0.1% Triton X-100 in 1X PBS at room temperature for 1 h. The antibodies were used at a 1:200 dilution.
9. 3 washes with 1X PBS were performed at room temperature.
10. The cells were left at 4°C in 1X PBS.

**Cardiomyocytic differentiation protocol with the Embryoid Bodies (EBs) formation step**

See protocol described in Ref. (57)

**CCD-camera based luminometer measurements with cardiomyocytes**

- EBs were dissociated at differentiation day 9 with Accutase™ (Chemicon, Cat. N. SCR005), counted and incubated (20000 cells/well in a 96 MTP) in 10 $\mu$M coelenterazine in standard tyrode buffer (for the test on GqPCRs) and in tyrode without KCl (for the test on L-type Calcium Channels) for 3 hrs at 37˚C.
- The tests were run 3 h after cell seeding.
- The responses to 50 nM Endothelin-1, 100 $\mu$M Noraepinephrine were measured at the CCD camera-based luminometer (conditions: high sensitivity, reading time 60 seconds).
  The Voltage gated Calcium Channels were stimulated with 40 mM KCl. The specificity of the channels was checked incubating the cells 15 minutes before he measurement with 5 $\mu$M Nifedipine (a specific L-type Voltage-gated Calcium Channel inhibitor) (SIGMA, Cat. N. N7634).
- Residual total photoprotein activity was measured after Triton X-100 cell lysis at the CCD camera-based luminometer (conditions: high sensitivity, for 30 seconds).

**Neuronal differentiation protocol with the Embryoid Bodies (EBs) formation step**

See protocol described in Ref. (64,65)

**CCD-camera based luminometer measurements with neurons**

- Retinoic Acid-induced (RA. Sigma, Cat. N. R-2625) EBs were dissociated and seeded 6500 cells/well in poly D-lysine (Sigma, Cat. N. G7121) coated 384 MTP and 24000 cells/well in 94 MTP format, for a further differentiation period.
  At differentiation day 14 they were incubated in 10 $\mu$M coelenterazine in standard tyrode buffer (for the test of Glutamate response) and in tyrode without KCl (for the test for Voltage-gated Calcium Channels, with or without a preincubation time of 15 minutes with 6 $\mu$M Omegaconotoxin GVIA (a specific N-type Voltage-gated Calcium Channel inhibitor) (BACHEM, Cat. ;N. H6615.1000) for 3 hrs at 37˚C.
- The responses were measured at the CCD camera-based luminometer (conditions for Glutamate response in 96 MTP: low sensitivity, reading time 60 seconds; conditions for Voltage-gated Calcium Channels response in 384 MTP: high sensitivity, reading time 60 seconds).

**Fluorometric Imaging Plate Reader (FLIPR®) measurements on differentiated neurons and undifferentiated ES / mito c-Photina® ES / 29 clone**

Differentiated cells (obtained after Retinoic Acid-induced EBs dissociation and cell seeding at 6500 cells/well on poly D-lysine coated 384 black wall clear bottom plates) were measured at day 16.

Undifferentiated ES / mito c-Photina® ES / 29 clone were seeded at 10000 cells/well 24 h before the test on gelatin coated 384 black wall clear bottom plates.

Before running the experiments, the medium was removed, and the cells were incubated in 25 $\mu$l/well Membrane Potential dye (Molecular Devices, Cat. N. R8034) solubilized in tyrode for 30 min at 37˚C.

12:5 $\mu$l/well of tyrode with 120 mM KCl (3X) (15 mM NaCl, 120 mM KCl, 2 mM CaCl$_2$, 5 mM NaHCO$_3$, 20 mM Hepes) were injected and the fluorescence signal was recorded for 250 sec and expressed as RFU (Relative Fluorescence Units).

FLIPR[384] settings:

   Exp. Time: 0.3 sec
   Injection speed: 20 $\mu$l/sec

Injection height: 50 $\mu$l

**Photoprotein tissue analysis on Photoprotein Transgenic Mice**

First experiment

Two mice, one positive and one negative for the c-Photina® transgene, were used. A sample of 200 $\mu$l of blood was withdrawn from tail veins of both mice. They were perfused with a physiological solution in order to eliminate blood contaminations. Several tissues were explanted from both mice *(brain, cerebellum, liver, fat, spleen, skeletal muscle, sciatic nerve, total pancreas, lung, kidney, blood, stomach, testis, heart),* and incubated with a solution containing 20 mM Tris-HCl pH7.5, 150 mM NaCl, 5 mM DTT, 1mM EDTA, 0.1% BSA, 20 $\mu$M coelenterazine plus protease inhibitor cocktails (Roche, Cat. N. 1836145), for 3 h at room temperature.

The samples were all cut with surgical scissors in order to reduce the tissue in smaller parts.

The samples were then aliquoted in 3 wells of a white 96 white well/plate.

They were all read at CCD camera-based luminometer, at high sensitivity, for 60 sec, at 0.6 sec of integration time, after injection of a solution of Triton X-100 and 250 mM CaCl$_2$.

In order to check the presence and the stability of the photoprotein reporter protein in the different tissues/ organs during time, 6 animals (3 positives and 3 negatives for the c-Photina® transgene) were further sacrificed at different ages: two mice 3 months old, two mice 6 months old, and two mice 10 months old. They were all perfused with a physiological solution in order to eliminate blood contaminations. Brain, cerebellum, spleen, lung, kidney, stomach, gonads, and heart were explanted from the mice, and incubated with a solution containing 20 mM Tris-HCl pH 7.5, 150 mM NaCl, 5 mM DTT, 1mM EDTA, 0.1% BSA, 20 $\mu$M coelenterazine plus protease inhibitor cocktails, for 3 h at room temperature.

The samples were all cut with surgical scissors in order to reduce the tissue in smaller parts, and then aliquoted in a white 96 white well/plate.

They were all read at CCD camera-based luminometer, at high sensitivity, for 60 sec, at 0.6 sec of integration time, after injection of a solution of Triton X-100 and 250 mM CaCl$_2$.

Second experiment

Two mice (one positive and one negative for the c-Photina® transgene) were used. A 300 $\mu$l of coelenterazine solution (373 $\mu$M coelenterazine, 3.3 % DMSO, 990 nM Glutathione in physiological solution), containing 2.8 mg of coelenterazine / kg of mouse, was injected via tail vein.

After 3 hrs a sample of 200 $\mu$l of blood was withdrawn from both mice tail veins.

- The mice were anesthetized with Avertin (12.5 $\mu$l/g body weight). Pancreatic islet isolation was performed from the mice by collagenase (type V, 1 mg/ml) (Sigma Chemical, St. Louis, MO) injection into hepatic duct. The mice were then sacrificed by exsanguination after a cut of the abdominal vena cava/aorta. The isolated pancreata were then digested and beta-islets were purified by density gradient (Histopaque-1077; Sigma). The islets were then cultured overnight at 37˚C in a humidified atmosphere with 5% CO$_2$ in M199 medium (Invitrogen, Cat. N. 22350-029), supplemented with 10% fetal calf serum, 100 U/ml penicillin, and 100 $\mu$g/ml streptomycin.
  10 Mito c-Photina® transgenic mice islets/well were put in a white 96 MTP and incubated in standard tyrode solution with 10 $\mu$M coelenterazine for 4 hrs at 37˚C. The islets calcium kinetics responses were measured at Luminoskan Ascent (Labsystems) luminomer; for 150 sec, at integration time 0.5 after stimulation with a glucose stimulus (11 mM), or with mannitol (11 mM), as negative control. The glucose concentration was then normalized at 3 mM and the islets were then stimulated with a depolarizing stimulus (40 mM KCl) at CCD camera-based luminomer (high sensitivity, for 60 sec). The total photoprotein content in the islets was measured after cell lyses with a Triton X-100-based buffer (high sensitivity, for 60 sec).
- Several tissues were then explanted from both mice *(brain, cerebellum, liver, fat, spleen, heart, sciatic nerve, stomach, lung, testis, bone, skeletal muscle, kidney, skin; thymus),* and cut with surgical scissors in order to reduce the tissues in smaller parts.

All the samples were divided in two batches. One part was put in a solution of 20 mM Tris-HCl pH7.5, 150 mM NaCl, 5 mM DTT, 1mM EDTA, 0.1% BSA, plus protease inhibitor cocktails without coelenterazine and tested immediately at the CCD camera-based luminometer; the other part was instead incubated with a solution containing 20 mM Tris-HCl pH7.5, 150 mM NaCl, 5 mM DTT, 1mM EDTA, 0.1% BSA, 20 $\mu$M coelenterazine plus protease inhibitor cocktails for 3 h at room temperature before the CCD camera-based luminometer test. All the samples were then aliquoted in 2 wells of a 96 white well/plate.

They were all read at CCD camera-based luminometer at high sensitivity, for 60 sec, at 0.6 sec of integration time, after injection of a solution of Triton X-100 and 250 mM $CaCl_2$.

Third experiment

One other c-Photina® transgenic mouse and one negative mouse were sacrificed in order to isolate monocytes from bone marrow (which were differentiated *in vitro* in macrophages) (68).
20000 cells/well were seeded in a 96 MTP plate for each mouse and cells were lysed with a solution of Triton X-100 in order to check the total cell lysis activity (high sensitivity, for 60 sec, integration time 0.6 see).

**P19 cell culture**

P19 Culture medium, seeding and incubation

P19 embryonic carcinoma pluripotent stem cells (ATCC, Cat. N. CRL-1825) are cultured with 10% Foetal Bovine Serum, FBS (ES qualified, Invitrogen, Cat. N. 16141079) αMEM, Minimum Essential. Medium Eagle with GLUTAMAX (Invitrogen, Cat. N. 32571028), 1% Pen./Strep. (Invitrogen, Cat. N.15140122) at 37˚C in a humidified atmosphere with 5% $CO_2$ (61).

Mito c-Photina® stable transfection

DNA construct was transfected using electroporation methods that can be replaced with a preferred protocol.
About 10 μg of the *mito c-Photina*® in pcDNA3 DNA was linearized with BglII (New England Biolabs) and was transfected by electroporation in $2.5\ 10^6$ cells (electroporation conditions: 500 μF, 0.24 kV, BioRad gene pulser).
The selection was started after 48 h from the transfection with 700 μg/ml G418. Colonies were generally ready for picking 8-9 days after electroporation.

Clone Selection Process:

1. P19 pcDNA3/ mito c-Photina® clones.
2. 24 h and 48 h after seeding, the transfected cells were plated in 2x96MTP white plates at 10000 and 15000 cells/well.
3. Medium was replaced with 50 μl/well of tyrode (2 mM $Ca^{2+}$ and coelenterazine 10 μM).
4. Positive clones were selected evaluating:

- Response to 100 μM Histamine. CCD camera-based luminometer conditions: high sensitivity, reading time 60 seconds.
- Residual total photoprotein activity measured after Triton X-100 cell lysis. CCD camera-based luminometer conditions: low sensitivity, for 30 seconds.

**P19 / mito c-Photina® / 1A1 clone *in vitro* neuronal differentiation**

See protocol described in Ref. (67)

**CCD-camera based luminometer measurements on neurons derived from P19 / mito c-Photina® / 1A1 Clone**

At day 8 and day 11 of the neuronal differentiation protocol (respectively 4 and 7 days after seeding in poly D-lysine coated 384 MTP), neurons differentiated from P19 / mito c-Photina® / 1A1 clone were incubated with 25 μl/well of 10 μM coelenterazine in standard tyrode buffer, for 4 hrs at 37˚C.
Response to a depolarizing stimulus induced by injection of 40 mM KCl was recorded at CCD camera-based luminometer (conditions: high sensitivity, reading time 60 seconds).
FLIPR® measurements on neurons derived from P19 / mito c-Photina® /1A1 clone
At day 8 of the neuronal differentiation protocol (4 days after seeding in poly D-lysine coated 384 well plates), neurons differentiated from P19 / mito c-Photina® / 1A1 clone were incubated with 25 μl/well of Fluo-4 NW® calcium sensitive fluorescent dye (Invitrogen, Cat. N. F36205) in the dark.
Plates were incubated for 30 minutes at 37˚C and then for other 30 minutes at room temperature.

12.5 μl/well of 120 mM KCl (3X) solution was injected, and the fluorescence signal was recorded for 330 sec and expressed as RFU (Relative Fluorescence Units).

FLIPR[384] settings:

Exp. Time: 0.3 sec
Injection speed: 20 μl/sec
Injection height: 50 μl
**FLIPR® measurements on undifferentiated P19 / mito c-Photina® / 1A1 clone and on neurons derived from P19 / mito c-Photina® / 1A1 clone**

Concerning undifferentiated P19 / mito c-Photina® / 1A1 clone, 3000 cells/well were seeded in gelatin coated 384 well plates. 24 h after seeding the cells were incubated with 25 μl/well of Fluo-4 NW® calcium sensitive fluorescent dye in the dark. Concerning neurons differentiated from P19 / mito c-Photina® / 1A1 clone, at day 8 of the neuronal differentiation protocol (4 days after seeding in poly D-lysine coated 384 well plates) cells were incubated with 25 μl/well of Fluo-4- NW® calcium sensitive fluorescent dye in the dark.
Both plates were incubated for 30 minutes at 37°C and then for 30 minutes at room temperature.
12.5 μl/well of the following 3X compounds in tyrode buffer were injected, and the fluorescence signal was recorded for 360 sec and expressed as RFU.
3X compounds: 3001 μM Histamine, and 300 μM Glutamate.

FLIPR[384] settings:

Exp. Time: 0.3 sec
Injection speed: 20 μl/sec
Injection height: 50 μl

Transient transfection Analysis:

1. 24 h after transfection medium was replaced with 50 μl/well of tyrode (2 mM $Ca^{2+}$ and coelenterazine 10 μM) and incubated for 4 h at 37°C.
2. 50 μl/well of 200 μM Histamine (2X) was injected using Luminoskan Ascent (Labsystems). Luminometer conditions: integration time 1 sec, reading time 60 seconds.

- Residual total photoprotein activity was measured after Triton X-100 cell lysis at Microlumat LB 96P (EG&G Berthold). Reading time 3 seconds.

Mito i-Photina® and mito Photina® stable transfection

DNA constructs were transfected using electroporation methods that can be replaced with a preferred protocol.
About 10 μg of *mito Photina*® and DNA were linearized with BglII (New England Biolabs) and were transfected by electroporation in 2.5 $10^6$ cells (electroporation condition: 500 μF; 0.24 kV, BioRad gene pulser).
The selections were started after 48 h from the transfection with 700 μg/ml G418. Colonies were pooled and collected 9 days after electroporation.

- Response to 50, 100 and 150 μM Histamine. CCD camera-based luminometer conditions: high sensitivity, reading time 60 seconds.
- Residual total photoprotein activity was measured after Triton X-100 cell lysis. CCD camera-based luminometer conditions: low sensitivity, for 30 seconds.

**Fluorometric Imaging Plate Reader (FLIPR®) measurements**

20000 cells/well were plated in 384 black wall clear bottom plates (MATRIX, Cat. N. 4332) (25 µl/well), the tests were run 24 h after cell seeding. Before running experiments the medium was removed, and cells were incubated in 50 µl/well Calcium 3 Assay kit 0.5X (Molecular Devices, Cat. N. R8090) for 30 min at 37˚C. 25 µl/well of Histamine 300 µM (3X) were injected and the fluorescence signal was recorded for 60 sec and expressed as RFU (Relative Fluorescence Units).

FLIPR[384] settings:

    Exp. Time: 0.2 sec
    Injection speed: 20 µl/sec
    Injection height: 50 µl

**EXAMPLES**

**1. Generation of ES cell line transfected with photoproteins**

**1.1.1 Mito c-Photina® ES TBV2 clone**

The murine ES TBV2 mito c-Photina® cell line was obtained by electroporation of ES TBV2 p16 cells with a pcDNA3 vector containing the *mito c-Photina*® photoprotein gene linearized with BglII restriction enzyme (Materials and Methods).
48 hours after transfection the cells were put in selection with 200 µg/ml G418. After 8 days of selection, 152 drug resistant colonies were picked. After morphological analysis only about 114 were expanded on MEF layers till they reach the confluence in 5 replicates in 96 well/plates of which:

    1- Two were on gelatin coated 96 white MTP for the test at the CCD camera-based luminometer at 24 and 48 h after seeding.
    2- One was on gelatin coated 96 white MTP for the DNA extraction.
    3- Two were on feeders layers for freezing and storage at -80˚C. **1.1.2 Mito c-Photina® ES TBV2 clone selection**

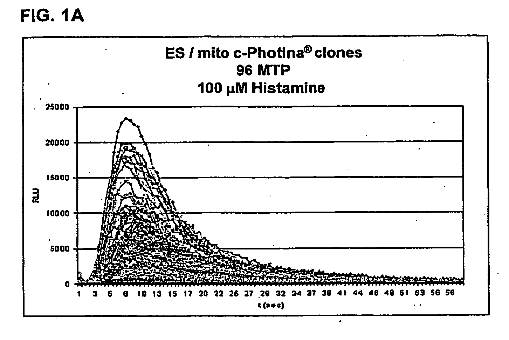

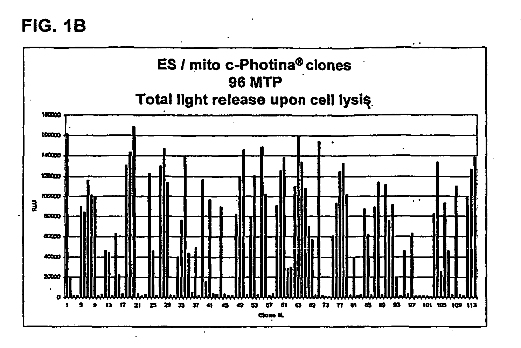

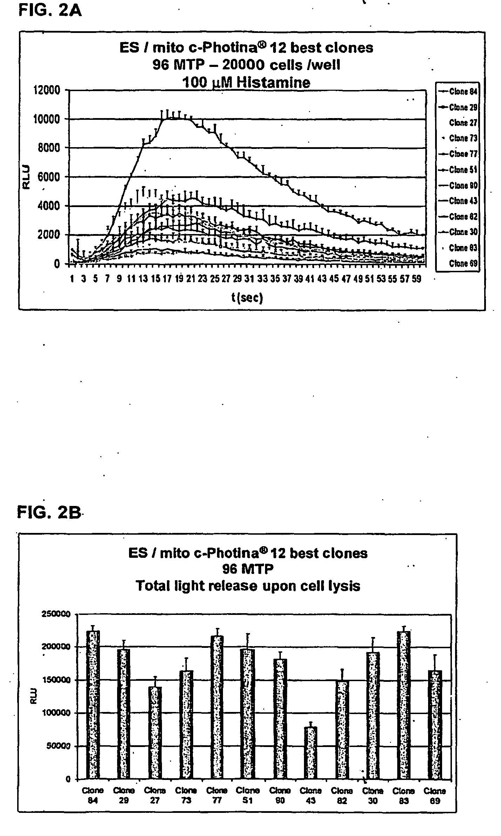

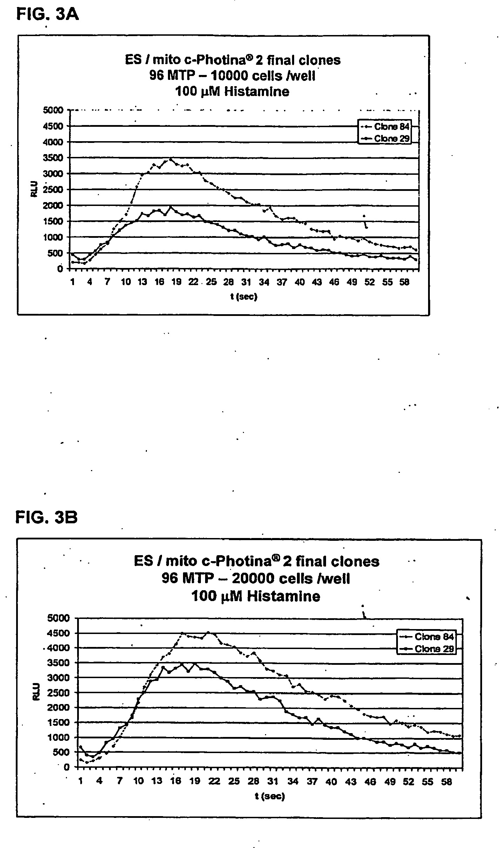

4 hours before measurement the medium of the positive clones was replaced with 50 µl/well of tyrode buffer 2 mM $Ca^{2+}$ and 10 µM coelenterazine in the dark, at 37˚C in a humidified atmosphere with 5% $CO_2$, in order to reconstitute the active photoprotein.
For light emission measurement, cells were first analyzed for the ability to respond (luminescent signal) to Histamine which is known to stimulate the ES endogenous Histamine -1 receptor (58) and to rise the cytoplasmic $Ca^{2+}$ concentration. The number of photons emitted after injection of 100 µM Histamine during the first 60 seconds was measured by a CCD camera-based luminometer. The kinetics of the response obtained is shown in Fig. 1A.
At the end of each experiment, cells were lysed (by a solution containing Triton X-100). All the photoprotein expressed in the cells react with free calcium and light emitted was measured (Fig.1B). The signal is an indicator of the total amount of photoprotein contained within the cells.
The 12 best Histamine responding clones were selected and retested at counted cells in 96 MTP (Fig. 2A and B).
The two final clones were selected on the basis of different parameters. The ability to respond to Histamine (Fig.3) and the total photoprotein content after cell lysis (data not shown) were the main discriminating factors, but also cell morphology and growth rate were analyzed. Furthermore Southern blot analysis (59) and Quantitative PCR (60) were performed. The Southern blot analysis was fundamental to ensure that only one random insertion occurred. To check this, the genomic DNA was digested with restriction enzymes that cut only one time in the vector transfected (to look for concatenates) or a double digestion with two enzymes (both used also in a single digestion) able to excide the photoprotein gene, assuring the specificity of the results. The probe used for the assay was the photoprotein gene. A quantitative PCR was also performed in order to analyze the number of gene insertion.
Final clones were also characterized by karyotype analysis (61). The clones N. 29 and 84 were selected. The clone N. 29 has only one photoprotein gene copy integrated in the genome, while the clone N. 84 has two copies as an inverted concatenate integrated only one time in the genome.
They were also differentiated into spontaneously beating cardiomyocytes after hanging drop Embryoid Bodies formation standard method and in neuronal cell types after Embryoid Bodies formation in presence of Retinoic Acid (57, 64, 65).

### 1.1.3. Stemness demonstration of ES / mito c-Photina® / 29 clone

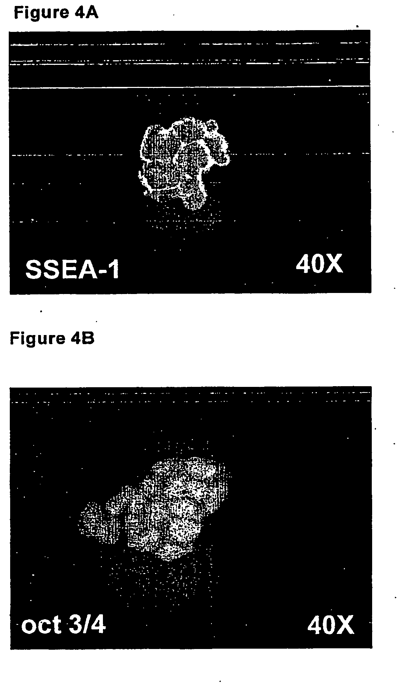

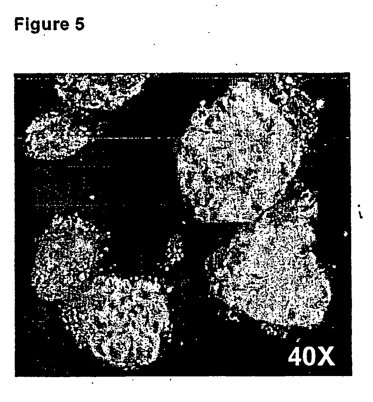

- An indirect iinmunofluorescence assays was performed on ES / mito c-Photina® / 29 clone in order to check the presence of specific markers of the undifferentiated pluripotent mouse embryonic stem cells, like the specific cells surface antigen (SSEA-1) (Fig. 4A) and the transcription factor **oct3/4** (Fig. 4B). The results were good and reported in figures 4A and 4B.
- Another stemness characteristic showed by these cells is the presence of the alkalin phosphatase enzyme activity measured with the ELF® Phosphatase staining kit (ATCC, Cat. N. SCRR-3010) (Fig. 5).

### 1.1.4. *In vitro* differentiation assays performed with ES / mito c-Photina® / 29 clone

The pluripotency of the 29 clone cells was also demonstrated by the ability of these cells to *in vitro* differentiate in cell types derived from different germ layers, like cardiomyocytes and neurons.

The differentiation experiments were performed using different approaches like the suspension protocols including the step of Embryoid Bodies (EB) formation and the protocols in adhesion (data not shown).

Optimal results were obtained in particular using protocols with the EBs step (see Material and Methods).

Cardiomyocytes

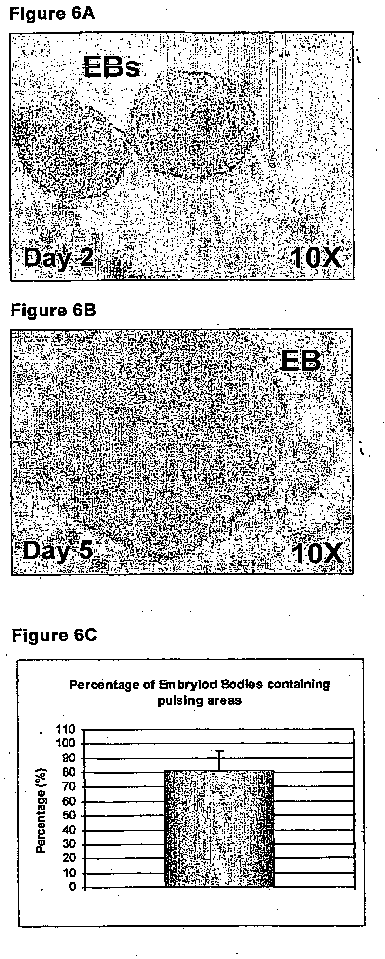

Using the procedure described above we saw the appearance of spontaneously pulsing cardiomyocytes starting from differentiation day 6. The percentage of EBs containing pulsing areas was about 80% (Fig. 6). This percentage was maintained also using different supports like i.e. gelatin-coated 24 MTP, 96 MTP, 384 MTP, and chamber slides (data not shown).

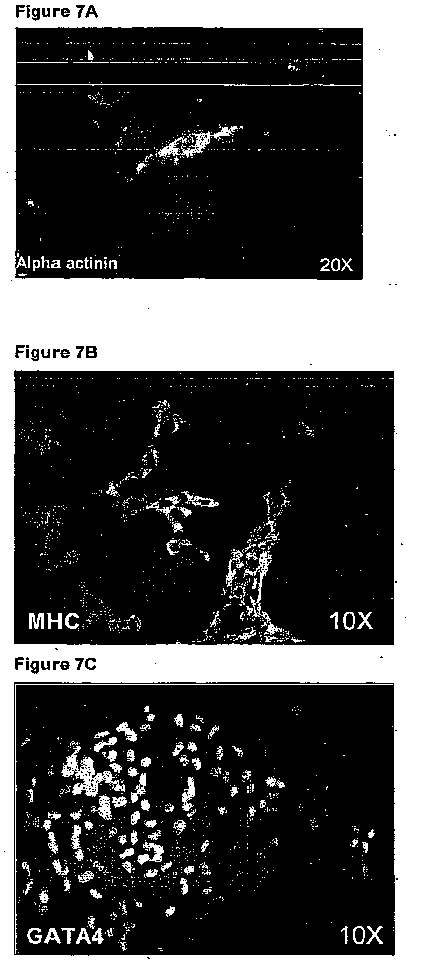

In order to verify the presence of mature cardiomyocytes an immunofluorescence assay was performed looking for the presence of specific cardiomyocytic markers like the cytoskeleton proteins alpha-actinin (Fig.7A), the Myosin Heavy Chain (MHC) (Fig. 7B), or the transcription factor GATA-4 (Fig. 7C)

Preliminary functional tests were performed at the CCD camera-based luminometer instrument after Embryoid Bodies disaggregation with Accutase® buffer and resuspension in 10 $\mu$M coelenterazine tyrode buffer. The cells were counted and seeded at a cellular concentration of 20,000 c/w in a 96MTP. After 4

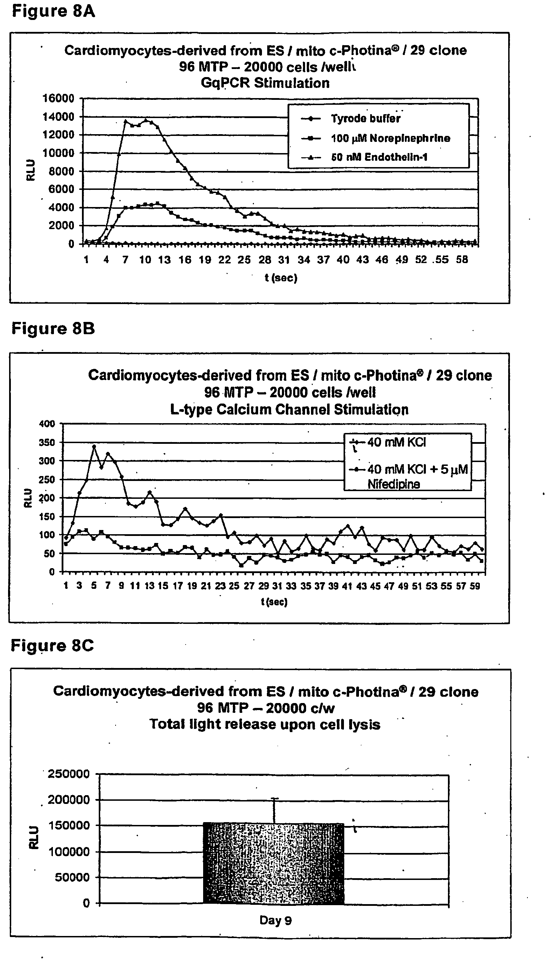

h at 37°C the cells were stimulated with standard tyrode buffer as control, 50 nM Endothelin-1 and 100 $\mu$M Norephinephrine, which are agonist respectively for the GqPCR Endothelin Receptors and for the $\alpha$1-Adrenergic Receptor, both present at high concentration in cardiomyocytes (CCD camera-based luminometer condition: high sens., for 60 sec). The responses showed were strong (most of all for Endothelin Receptor) and specific (Fig.8A). The same cells were stimulated with 40 mM KCl, a depolarizing stimulus, in order to activate the L-type Voltage Gated Calcium Channels, present at high concentration in cardiomyocytes. The specificity of the responses were investigated preincubating or not for 15 min the cells (before the KCl injection) with 5 $\mu$M Nifedipine, which is a specific L-type Voltage Gated Calcium Channel inhibitor (Fig. 8B).

The residual photoprotein activity was checked injecting a cell lysis buffer (CCD camera-based luminometer condition: high sens., for 30 sec) (Fig. 8C).

Neurons

For the neuronal differentiation the Embryoid Bodies were formed in presence of 1$\mu$M of all trans Retinoic Acid. 2 days after the plating on tissue culture treated dishes it was visible the presence of cellular prolongations whose length increase with time (Fig. 9).

The EBs Retinoic Acid-treated can be also disaggregated, replated on different coating substrates and cultured with neuronal specific media in absence of serum. In these cells the presence of specific markers was investigated by immunoflurescence (neurofilament H, neuronal nuclei antigen, here reported on double staining with two different fluorocromes - Fig. 10A and B), or glial markers (glial fibrillary acidic protein. Fig. 10C), or markers for neural precursors cells (nestin. Fig. 10D). It is important to note that this kind of cell population is not made by fully differentiated neurons but contain also glial cells and neural precursors. In figure 10 is reported an example of such a population (at differentiation day 18).

The functionality of these cells was investigated at CCD camera-based luminometer. At differentiation day 14, the cells were incubated for 4 h with a tyrode solution containing 10 $\mu$M coelenterazine. The cells were stimulated

injecting of 100 μM Glutamate (Fig. 11A) for investigating the Glutamate Receptor response, or depolarized with 40 mM KCl in presence or in absence of 6 μM Omegaconotoxin GVIA (preincubated for 15 minutes) in order to specific inhibit the N-type Voltage-gated Calcium Channels (Fig. 11B).

The functionality of these cells was also investigated at FLIPR[384]. At differentiation day 16 the medium was removed and cells incubated with membrane potential dye solubilised in standard tyrode for 30 min at 37˚C. After stimulation with 40 mM KCl, the fluorescent signal was recorded for 180 sec and expressed as RFU (Fig. 12, continuous line). The same experiment was performed on undifferentiated ES / mito c-Photina® / 29 clone, as control (Fig. 12, dashed line).

### 1.1.5. Germline transmission analysis

The mouse embryonic stem cells (ES TBV2) containing the photoprotein reporter gene was tested by germline transmission. Clones N. 29 and 84 were both injected into blastocysts of pregnant host female mice (EMBL Monterotondo). The progenies showed a high degree of chimerism (almost 100%) and male phenotypes.

The 2 best chimeric male mice derived from 29 were selected, and when they reached the sexual maturity, were crossed with BL6 female mice to investigate the germline transmission ability of the transgenic ES cells. The germline transmission is the only incontrovertible way to demonstrate the totipotency of the mouse embryonic stem cells. As expected, half of the mice born from these crosses are transgenic mice heterozygous for the photoprotein gene.

These heterozygous transgenic mice were crossed themselves in order to obtain a homozygous population. One fourth of the born mice were homozygous and phenotypically normal, demonstrating that the transgene did not disrupt any crucial gene.

These c-Photina® transgenic mice are a very precious source of cells as the adult stem cells (for example haematopoietic, or mesenchymal stem cells), committed progenitors, and also primary cells containing the photoprotein.

The cells derived from photoprotein transgenic animals can be used as positive controls for the "primary-like" cells (obtained after differentiation from the ES cells), but they are also a good source of photoprotein containing primary cells, for the HTS process *per se.*

For this reason we decided to investigate in which tissues the photoprotein was expressed.

We sacrificed two mice, one positive and one negative for the c-Photina® transgene. Several tissues were explanted from both mice, and incubated with a solution containing 20 mM Tris-HCl pH7.5, 150 mM NaCl, 5 mM DTT, 1 mM EDTA, 0.1 % BSA, 20 μM coelenterazine plus protease inhibitor cocktails. After 3 h of incubation at room temperature we lysed the tissues/organs injecting a solution of Triton X-100 in contemporary to 250 mM $CaCl_2$, in order to release all the photoprotein present in the samples in a not saturating calcium environment (Fig. 13A). The presence of the photoprotein transgene in the different tissues was checked during time in animals of different ages (3, 6, 10 months old) contemporary analyzing more animals with the same procedures described above. Measured signals seem to be stable during time holding account of the fact that the samples amount was not normalized (Fig. 13B).'

Then we performed a second experiment in which we checked the ability of the coelenterazine to diffuse and charge the photoprotein present in the different tissues/organs after intravenously systemic injection of 2.8 mg of coelenterazine /kg via tail vein (66). After 3 hours the mice were sacrificed and several tissue/organs explanted. Half of the material was directly tested at the CCD camera-based luminometer after cell lysis and injection of a calcium solution (high sensitivity, for 60 seconds, 0.6 integration time) (Fig. 14A). The other half of the material was transferred to another 96 MTP and incubated for other 3 h with a solution containing 20 μM coelenterazine. Also this other plate was tested at the CCD camera-based luminometer after cell lysis and injection of a calcium solution (high sensitivity, for 60 seconds, 0.6 integration time) (Fig. 14B).

Before the sacrifices, we performed also a pancreatic islet isolation and purification (see material and methods). Islets were cultured overnight at 37˚C. The day after the islets were manually picked and put in 96 MTP (10 islets/well). They were incubated in standard tyrode containing 10 μM coelenterazine for 3 h at 37˚C.

After that time they were stimulated with 11 mM glucose, in order to activate the calcium-mediated insulin pathway. As control the islets were stimulated also with another sugar (which is not able to induce the calcium-mediated insulin response), mannitol (at 11 mM final concentration). They were measured at Luminoskan luminomer; for 150 sec, at integration time 0.5 (Fig. 15A).

The glucose concentration was then normalized at 3 mM and the islets were furthermore stimulated with a depolarizing agent (40 mM KCl), and measured at CCD camera-based luminometer (high sensitivity, for 60 seconds) (Fig. 15B). The residual photoprotein activity was checked injecting a cell lysis buffer (high sensitivity, for 60 seconds) (Fig. 15C).

The transgenic animals are also a very important source of primary cells containing the photoprotein. Fot this purpose, as example of primary cells, monocytes were isolated from the bone marrow of a positive and a negative mouse as control. These cells were then *in vitro* differentiated in order to obtain macrophages (68). After the establishment of the cell culture, the presence of the c-Photina® transgene was checked lysing the cells with a solution of Triton X-100 (Fig. 16).

### 1.2.1. Mito i-Photina® ES TBV2 clone

The murine ES TBV2 mito i-Photina® cell line was obtained by electroporation of ES TBV2 p16 cells with a pcDNA3 vector containing the *mito i-Photina®* photoprotein gene linearized with BglII restriction enzyme (Materials and Methods).

48 hours after transfection the cells were put in selection with 200 μg/ml G418. After 8 days of selection, 130 drug resistant colonies were picked and expanded on MEF layers till they reach the confluence in 5 replicates in 96 well/plates of which:

- Two were on gelatin coated 96 white MTP for the test at the CCD camera-based luminometer at 24 and 48 h after seeding.
- One was on gelatin coated 96 white MTP for the DNA extraction.
- Two were on feeders layers for freezing and storage at -80°C.

### 1.2.2. Mito i-Photina® ES TBV2 clone selection

The clones were selected exactly as reported above for ES TBV2 mito c-Photina® cell line. The final clones are the numbers 70 and 43. The 70 clone showed the highest Histamine response (Fig. 17A), but also the clone 43 showed good histamine-dose response (data not shown). The total light emission upon cell lysis is good for both (Fig. 17B).

They were analyzed also by Southern blot showing only one integration, but not for Real Time PCR.

The karyotype for both clones was correct.

### 1.3.1. i-Photina® ES TBV2 clone

The murine ES TBV2 i-Photina® cell line was obtained by electroporation of ES TBV2 p16 cells with a pcDNA3 vector containing the *i-Photina®* photoprotein gene linearized with BglII restriction enzyme (Materials and Methods).

48 hours after transfection the cells were put in selection with 200 μg/ml G418. After 8 days of selection, 99 drug resistant colonies were picked and expanded on MEF layers till they reach the confluence in 5 replicates in 96 well/plates of which:

- Two were on gelatin coated 96 white MTP for the test at the CCD camera-based luminometer at 24 and 48 h after seeding.
- One was on gelatin coated 96 white MTP for the DNA extraction.
- Two were on feeders layers for freezing and storage at -80°C.

### 1.3.2. i-Photina® ES TBV2 clone selection

The clones were selected exactly as reported above for ES TBV2 mito c-Photina® cell line. The final clones are the numbers 113 and 109. The 113 clone showed the highest Histamine response (Fig. 18A). The total light emission upon cell lysis is good for both (Fig. 18B).

They were analyzed also by Southern blot showing only one integration, but not for Real Time PCR.

The karyotype for both clones was correct.

### 2. Generation of P19 cell line transfected with photoproteins

### 2.1.1. Mito c-Photina® P19 clone

The P19 mito c-Photina® cell line was obtained by electroporation of P19 cells with a pcDNA3 vector containing the *mito c-Photina®* photoprotein gene linearized with BglII restriction enzyme (Materials and Methods).

48 hours after transfection the cells were put in selection with 700 μg/ml G418. After about 7-8 days of selection, drug resistant colonies were picked, and expanded.

### 2.1.2. Mito c-Photina® P19 selection

4 hours before measurement the medium was replaced with 50 μl/well of tyrode buffer 2 mM $Ca^{2+}$ and 10 μM coelenterazine in the dark, at 37°C in a humidified atmosphere with 5% $CO_2$, in order to reconstitute the active photoprotein.

For light emission measurement, cells were first analyzed for the ability to respond (luminescent signal) to Histamine which is known to stimulate the P19 endogenous Histamine -1 receptor (58) and to rise the cytoplasmic $Ca^{2+}$ concentration. Two final clones were selected on the basis of the photoprotein activity in response to Histamine and on the photoprotein total content measured after cell lysis with Triton X-100 (Fig.19). It was also considered their ability to differentiate into spontaneously beating cardiomyocytes and neurons after differentiation process obtained using Embryoid Bodies formation in presence or absence of 0.5-1% DMSO as inducing agent for cardiomyocytes development and Retinoic Acid for neural one (61,62,64,67).

**2.1.3. P19 / mito c-Photina® / 1A1 Clone *in vitro* differentiation toward the neuronal lineage**

The 1A1 clone of pluripotent embryonic carcinoma P19 expressing mito c-Photina® cells was shown to be able to differentiate *in vitro* in neuronal cell types.

It was demonstrated by immunofluorescence that these cells expressed neuronal specific markers like Neurofilament H (NF H) and Neuronal Nuclei (NeuN) (Fig. 20A and 20B), or neural precursors cells markers (Nestin) (Fig.20C).

The functionality of these cells was also investigated at CCD camera-based luminometer . At differentiation day 8 and 11 (respectively 4 and 7 days after disaggregation of Embryoid Bodies and seeding on poly D-lysine in 384 MTP), the cells were incubated for 4 h with a tyrode solution containing 10 $\mu$M coelenterazine. Voltage-gated Calcium Channels were stimulated with injection of 40 mM KCl, or with standard tyrode as control, showing optimal responses (Fig. 21).

At differentiation day 8 the same cells were analyzed also at FLIPR[384] incubating the cells with FLUO-4 NW® and injecting 40 mM KCl depolarizing agent. Also in this case there is a sensible increase of the signal due to an entrance of calcium in the cell, even if with worst kinetics shapes then those observed with the luminescence reporter system (Fig. 22).

These cells at the same developmental stage (day 8) were analyzed at FLIPR[384] also for the presence of Metabotropic or-Ionotropic Glutamate Receptors (Fig. 23B, dashed line) or for Histamine-1 Receptor (GqPCR) (Fig. 23B, continuous line). The same experiment was in contemporary performed on undifferentiated P19 / mito c-Photina® 1A1 clone (at 24 h after seeding of 3000 cells/well in 384 MTP. Fig. 23A). Notably, the signal recorded after Histamine stimulation was higher in the undifferentiated cells (as expected since it is highly expressed at that stage) and decrease during differentiation. The contrary was shown for the Glutamate stimulation since it is much higher in the differentiated cells than in the undifferentiated ones. The presence of Glutamate Receptors is a further demonstration of the neuronal commitment of these cells.

**3. Transient transfections**

The P19 cells were transfected with different mitochondrial tagged photoproteins (materials and methods) to evaluate the ability of these other photoproteins to measure intracellular calcium release and to obtain information on the photoprotein expression levels.

4 hours before measurement the medium was replaced with tyrode buffer and 10 $\mu$M coelenterazine in the dark, at 37˚C in a humidified atmosphere with 5% $CO_2$, in order to reconstitute the active photoprotein.

The luminescence signal was recorded for 60 seconds after 100 $\mu$M Histamine injection.

The cells were then lysed with Triton X-100 in order to detect the total light release (Fig.24).

**4. Stable transfections**

Stable transfections of the different mitochondrial tagged photoprotein in P19 cells (materials and methods) were performed in order to investigate the levels of photoprotein expression in a stably integrated manner and to verify that none of the photoproteins stably expressed in P19 cells are toxic over a long period of time in culture. 4 hours before measurement the medium was replaced with tyrode buffer and 10 $\mu$M coelenterazine in the dark, at 37˚C in a humidified atmosphere with 5% $CO_2$, in order to reconstitute the active photoprotein.

The luminescence signal was recorded after 50, 100 and 150 $\mu$M Histamine injection and measured for 60 seconds.

The cells were then lysed with Triton X-100 in order to detect the total light release (Fig.25).

**5. Cell-based fluorescence assays at the FLIPR[384]**

The P19 mito c-Photina® final clones (1A1 and 1A2) were tested also at FLIPR[384] by measuring the calcium concentrations variation induced by the activation of the endogenous Histamine 1 receptor with a detection method that uses fluorescence instead of luminescence.

The cells were incubated with the Calcium 3 assay kit (Molecular Devices Corporation, Sunnyvale, CA, USA).

These experiments were carried out to compare the results obtained using fluorescence calcium detection methods instead of luminescence-based calcium detection and to evaluate the advantages of luminescence over fluorescence. The results obtained show that fluorescence-based method has a higher background compare to luminescence. This is reflected in a lower signal to noise background of fluorescence. On the contrary, the signal to noise background for luminescence is higher and this reflects in a wider dynamic range compare to fluorescence (Fig.26).

## REFERENCES

1. Burdon T., Smith A., Savatier P. (2002) Signalling, cell cycle and pluripotency in embryonic stem cells. Trends Cell Biol.; 12(9), 432-8.

2. Odorico J.S., Kaufman D.S., Thomson JA. (2001) Stem Cells. Multilineage differentiation from human embryonic stem cell lines.; 19(3), 193-204.

3. Wobus, AM and Boheler, KR. (2005) Embryonic stem cells: prospects for developmental biology and cell therapy. Physiological Reviews; 85, 635-678.

4. Spangrude, G. J., Heimfeld, S. and Weissman, I. L. (1988) Purification and characterization of mouse hematopoietic stem cells. Science; 241, 58-62.

5. Krause D.S., Theise N.D. Collector M.I., Henegariu O., Hwang S., Gardner R., Neutzel S., Sharkis S.J. (2001) Multi-organ, multi-lineage engraftment by a single bone marrow derived stem cell. Cell; 105, 369-377.

6. Reya T., Sean J. Morrison S.J., Clarke M.F.3 and Weissman I.L. (2001) Stem cells, cancer, and cancer stem cells. Nature; 414, 105-111.

7. Wagers A.J, Weissman I.L. (2004) Plasticity of adult stem cells. Cell; 116(5), 639-648.

8. Baksh D., Song L., Tuan R.S. (2004) Adult mesenchymal stem cells: characterization, differentiation, and application in cell and gene therapy. J Cell Mol Med.; 8(3), 301-316.

9. Cai J., Weiss M.L., Rao MS (2004) In search of "stemness". Exp Hematol.; 32(7), 585-598.

10. Donovan P.J., and Gearhart J. (2001) The end of the beginning for pluripotent stem cells. Nature; 414, 92-97.

11. Evans M.J., Kaufman M.H. (1981) Establishment in culture of pluripotential cells from mouse embryos. Nature; 292(5819), 154-156.

12. Martin G.R. (1981) Isolation of a pluripotent cell line from early mouse embryos cultured in medium conditioned by teratocarcinoma stem cells. Proc Natl Acad Sci U S A.; 78(12), 7634-7638.

13. Thomson J.A., Itskovitz-Eldor J., Shapiro S.S., Waknitz M.A., Swiergiel J.J., Marshall V.S., Jones J.M. (1998) Embryonic stem cell lines derived from human blastocysts. Science; 282(5391), 1145-1147.

14. Stevens, L. C. (1967) The biology of teratomas. Adv. Morphogen.; 6, 1-31.

15. Gearhart J.D., Mintz B. (1974) Contact-mediated myogenesis and increased acetylcholinesterase activity in primary cultures of mouse teratocarcinoma cells. Proc Natl Acad Sci U S A.; 71(5), 1734-1738.

16. Kahan B.W., Ephrussi B.J. (1970) Developmental potentialities of clonal in vitro cultures of mouse testicular teratoma. Natl Cancer Inst.; 44(5),1015-1036.

17. Jiang, L. I. and Nadeau, J. H. (2001) 129/Sv mice--a model system for studying germ cell biology and testicular cancer. Mammal. Genome 12, 89-94.

18. McBurney M.W. (1993) P19 embryonal carcinoma cells. Int J Dev Biol. Mar; 37(1), 135-140.

19. Matsui, Y., Zsebo, K. and Hogan, B. L. (1992) Derivation of pluripotential embryonic stem cells from murine primordial germ cells in culture. Cell; 70, 841-847.

20. Labosky P.A., Barlow D.P., Hogan B.L. (1994a) Embryonic germ cell lines and their derivation from mouse primordial germ cells. Ciba Found Symp.;182,157-68; discussion 168-178.

21. Labosky P.A., Barlow D.P., Hogan B.L. (1994) Mouse embryonic germ (EG) cell lines: transmission through the germline and differences in the methylation imprint of insulin-like growth factor 2 receptor (Igf2r) gene compared with embryonic stem (ES) cell lines.Development; 120(11), 3197-3204.

22. Resnick JL, Bixler LS, Cheng L, Donovan PJ. (1992) Long-term proliferation of mouse primordial germ cells in culture. Nature; 359(6395),550-551.

23. Bradley A., Evans M., Kaufman M.H., and Robertson E. (1984) Formation of germ-line chimeras from embryo-derived teratocarcinoma cell lines. Nature; 309, 255-256.

24. Stewart C.L., Gadi I., and Bhatt H. (1994) Stem cells from primordial germ cells can reenter the germ line. Dev. Biol.; 161, 626-628.

25. Brinster, R.L. (1974) The effect of cells transferred into the mouse blastocyst on subsequent development.J. Exp. Med. 140, 1049-1056.

26. Illmensee K., and Mintz B. (1976) Totipotency and normal differentiation of single teratocarcinoma cells cloned by injection into blastocysts. Proc. Natl Acad. Sci. USA; 73, 549-553.

27. Papaioannou V.E., Gardner R.L., McBurney M.W., Babinet, C. and Evans M.J. (1978) Participation of cultured teratocarcinoma cells in mouse embryogenesis. J. Embryol. Exp. Morphol.; 44, 93-104.

28. Martin G.R. (1980) Teratocarcinomas and mammalian embryogenesis. Science 209, 768-776.

29. McNeish J. (2004) Embryonic stem cells in drug discovery. Nat Rev Drug Discov.; 3(1), 70-80.

30. Seiler A., Visan A., Buesen R., Genschow E. and Spielmann H.(2004) Improvement of an in vitro Stem Cell Assay for developmental toxicity: the use of molecular endpoints in the embryonic stem cell test. Reproductive Toxicology, 18, 231-240.

31. Genschow E., Spielmann H., Scholz G., Seiler A., Brown N., Piersma A., Brady M., Clemann N., Huuskonen H., Paillard F., Bremer S., and Becker K. (2002) The ECVAM international validation study on in vitro embryotoxicity tests: results of the definitive phase and evaluation of prediction models. European Centre for the Validation of Alternative Methods. Altern Lab Anim.; 30(2), 151-76.

32. Genschow E., Spielmann H., Scholz G., Pohl I., Seiler A., Clemann N., Bremer S., and Becker K. (2004) Validation of the embryonic stem cell test in the international ECVAM validation study on three in vitro embryotoxicity tests. Altern Lab Anim.; 32(3), 209-244.

33. Kendall J.M., and Badminton M.N. (1998) Aequorea victoria bioluminescence moves into an exciting new era. Trends Biotechnology; 16(5), 216-224.

34. Campbell A.K., Hallet, R.A., Daw, M.E., Ryall, R.C., Hart, and Herring P.J. (1981) Application of the photoprotein obelin to the measurement of free Ca++ in cells. In Bioluminescence and Chemiluminescence, basic Chemistry and Analytical applications (Edited by M.A. deLuca and W.D. McElroy), pp. 601-607. Academy Press, New York.

35. Herring P.J. (1979) Some features of the bioluminescence of the radiolarian Thalassicola sp. Mar. Biol.; 53, 213-216.

36. Shimomura O., Johnson F.H., and Saiga Y. (1962) Extraction, purification and properties of aequorin, a bioluminescent protein from the luminous hydromedusan, Aequorea. J. Cell. Comp. Physiol.; 59, 223-239.

37. Shimomura O., Johnson F.H., and Saiga, Y (1963) Further data on the bioluminescent protein, aequorin. J. Cell. Comp. Physiol.; 62, 1-8.

38. Morin J.G. and Hastings J.W. (1971) Biochemistry of the bioluminescence of colonial hydroids and other coelenterates. J. Cell. Physiol.; 77, 305-311.

39. Shimomura O., Johnson F.H. and Saiga, Y. (1963) Extraction and properties of halistaurin, a bioluminescent protein from the hydromedusan Halistaura. J. Cell. Physiol.; 62, 9-15.

40. Shimomura O., and Shimomura A (1985) Halistaurin, phialidin and modified forms of aequorin as Ca++ indicator in biological systems. Biochem. J.; 228, 745-749.

41. Levine L.D., and Ward W.W. (1982) Isolation and characterization of a photoprotein "phialidin" and a spectrally unique green-fluorescent protein from the bioluminescent jellyfish Phialidium gregarium. Comp. Biochem. Physiol.; 72B, 77-85.

42. Morin J.G. and Hastings J.W. (1971) Energy transfer in a bioluminescent system. J. Cell. Physiol. 77, 313-318.

43. Campbell A.K. (1974) Extraction, partial purification and properties of obelin the calcium-activated protein from the hydroid Obelia geniculata. Biochem.J.; 143, 411-418.

44. Ward W.W. and Selinger H.H. (1974) Extraction and purification of calcium-activated photoprotein from the ctenophores Mnemiopsis sp. and Bern ovata. Biochemistry; 13, 1491-1499.

45. Ward W.W. and Seliger H.H. (1974) Properties of mnemiopsin, and berovin, calcium-activated photoproteins from the ctenophores Mnemiopsis sp. and Beroë ovata. Biochemistry 13, 1500-1510.

46. Johnson F.H. and Shimomura O. (1978) Introduction to the bioluminescence of medusae, with special reference to the photoprotein aequorin. Methods Enzymol.; 57, 271-291.

47. Illarionov B.A., Bondar V.S., Illarionova V.A., Vysotski E.S. (1995) Sequence of the cDNA encoding the Ca++-activated photoprotein obelin from the hydroid polyp Obelia longissima. Gene; 14;153(2), 273-274.

48. Blinks J.R., Weir W.G., Hess P. and Prendergast F.G. (1982) Measurement of Ca++ concentrations in living cells. Prog. Biophys. Mol. Biol.; 40,1-114.

49. Markova S.V., Vysotski E.S., Blinks J.R., Burakova L.P., Wang B.C., Lee J., (2002) Obelin from the bioluminescent marine hydroid Obelia geniculata: cloning, expression, and comparison of some properties with those of other Ca2+-regulated photoproteins. Biochemistry; 41(7),2227-36.

50. Inouye S., Tsuji F.I. (1993) Cloning and sequence analysis of cDNA for the Ca(2+)-activated photoprotein, clytin. FEBS Lett.; 315(3), 343-346.

51. Tsuji F.I., Ohmiya Y., Fagan T.F., Toh H., Inouye S. (1995) Molecular evolution of the Ca(2+)-binding photoproteins of the Hydrozoa. Photochem Photobiol.; 62(4), 657-661.

52. Rizzuto R., Simpson A.W.M., Brini M. and Pozzan T. (1992) Rapid changes of mitochondrial Ca2+ revealed by specifically targeted recombinant aequorin. Nature; 358, 325-328.

53. Rizzuto R., Brini M., Murgia M. and Pozzan T. (1993) Microdomains with high Ca2+ close to IP3-sensitive channels that are sensed by neighbouring mitochondria. Science; 262, 744-747.

54. Rizzuto R., Bastianutto C., Brini M, Murgia M. and Pozzan T. (1994) Mitochondrial Ca2+ homeostasis in intact cells. J. Cell Biol.; 126, 1183-1194.

55. Nagy A., Gertsenstein M., Vinterstein K., Behringer R. (2003) Manipulating the mouse embryo. A laboratory manual. Third edition. Cold Spring Harbor Laboratory Press.

56. Turksen K.(2002) Embryonic Stem Cells. Methods and protocols. Methods in Molecular Biology. Vol 185. Humana Press

57. Boheler K.R. (2003) ES cell differentiation to the cardiac lineage. Methods in enzymology; 365, 228-241.

58. Bloemers S.M., Leurs R., Smit M.J., Verheule S., Tertoolen L.G.J., Timmerman H., and de Laat S.W. (1993) Mouse P19 embryonal carcinoma cells express functional Histamine H1-receptors. Biochemical and Biophysical Research' Communications; 191, 118-125.

59. Sambrook J., and Russel D. W. (2001) Molecular cloning. A laboratory manual. Third edition. Cold Spring Harbor Laboratory Press.

60. Ramakers C., Ruijter J.M., Deprez R.H., Moorman A.F. (2003) Assumption-free analysis of quantitative real-time polymerase chain reaction (PCR) data. Neuroscience Letters; 339, 62-66.

61. Rudnicki M.A. and McBurney M.W. (1987) Cell culture methods and induction of differentiation of embryonal carcinoma cell lines. In: E.J. Robertson (Ed.), Teratocarcinomas and Embryonic Stem Cells: A Practical Approach, IRL Press, Oxford, pp. 19-49.

62. J. Paquin B. Danalache M. Jankowski S.M., McCann J., Gutkowska J. (2002) Oxytocin induces differentiation of P19 embryonic stem cells to cardiomyocytes. Proc. Nat. Acad. Sci. USA; 99, 9550-9555.

63. Bolino A., Bolis A., Previtali S.C., Dina G., Bussini S., Dati G., Amadio S., Del Carro U., Mruk D.D., Feltri M.L., Cheng C.Y., Quattrini A., Wrabetz L. (2004). Disruption of Mtmr2 produces CMT4B1-like neuropathy with myelin outfolding and impaired spermatogenesis. J Cell Biol; 167, 711-721.

64. Bain G., Kitchens D., Yao M., Huettner J.E., Gottlieb D.I. (1995). Embryonic stem cells express neuronal properties in vitro. Dev Biol; 168, 342-357.

65. Okada Y., Shimazaki T., Sobue G., Okano H. (2004). Retinoic-acid-concentration dependent acquisition of neural cell identity during in vitro differentiation of mouse embryonic stem cells. Dev Biol 275: 124-142.

66. Bhaumik S., Gambhir S.S. (2002). Optical imaging of Renilla luciferase reporter gene expression in living mice. PNAS; 8;99(1):377-82.

67. Maltais D., Desroches D., Aouffen M., Mateescu M.A., Wang R., Paquin J. (2003). The blue copper ceruloplasmin induces aggregation of newly differentiated neurons: a potential modulator of nervous system organization. Neuroscience;121(1):73-82.

68. Davies J. Q., Gordon S. (2005). Isolation and culture of murine macrophages Meth. in Mol. Biol. Vol 290: Basic Cell Culture Protocols, Third Edition; 91- 103.

**Claims**

1. A non human transgenic animal, the progeny and cell derivatives thereof able to express an apophotoprotein gene and to produce a bioluminescent signal in response to intracellular calcium concentration variation, wherein the apophotoprotein gene expression and/or the production of a bioluminescent signal in response to intracellular calcium concentration variation is ubiquitous.

2. The non human transgenic animal, the progeny and cell derivatives thereof according to claim 1 wherein the ubiquitous apophotoprotein gene expression and/or the production of a bioluminescent signal in response to intracellular calcium concentration variation is inducible.

3. The non human transgenic animal, the progeny and cell derivatives thereof according to claim 1 or 2 wherein the apophotoprotein gene is selected from the group comprising:

- the apophotoprotein sequence of SEQ ID No. 1,
- the apophotoprotein sequence of the Clytin sequence (GenBank accession number Q08121) mutagenised in the following position Gly142->Cys, or
- the apophotoprotein sequence of the Clytin sequence (GenBank accession number Q08121) mutagenised in the following 12 positions: Gry58->Glu, Asp69->Val, Ala70->Cys, Lys76->Arg, Lys77->Gly, Ile78->Cys, Asp81->Glu, Va186->Ile, Glu87->Ala, Ala90->Gln, Va192-->Leu, and Glu97->Gln.

4. The non human transgenic animal, the progeny and cell derivatives thereof according to any of previous claims being a non human mammal.

5. The non human transgenic animal, the progeny and cell derivatives thereof according to claim 4 being a rodent.

6. The non human transgenic animal, the progeny and cell derivatives thereof according to claim 5 being a mouse.

7. The non human transgenic animal, the progeny and cell derivatives thereof according to any of previous claims wherein the apophotoprotein gene is any apophotoprotein gene, of natural or recombinant or synthetic origin.

8. The non human transgenic animal, the progeny and cell derivatives thereof according to claim 7 wherein the apophotoprotein is a natural or mutagenized mutant having an improved luminescent activity and/or calcium sensibility.

9. The non human transgenic animal, the progeny and cell derivatives thereof according to claim 8 wherein the apophotoprotein gene has a sequence optimized for mammalian codon usage and/or fused to mitochondrial target sequences.

10. A cell derivative of the non human transgenic animal according to any of previous claims being able, or being differentiated into a specific cell lineage, to get expression of a specific target gene.

11. The cell derivative of the non human transgenic animal according to claim 10 wherein the specific cell lineage is the muscle heart cell lineage or the neuronal lineage or the mesenchymal cell lineage or the haematopoietic cell lineage.

12. A method for identifying a ligand able to stimulate a specific target so that a variation of intracellular $Ca^{++}$ is obtained comprising the steps of:

    a) providing a cell derivative according to claims 10 or 11;
    b) getting expression of said target;
    c) loading cells with a suitable chromophore as substrate;
    d) contacting cells with a compound library comprising putative ligands for said target;
    e) detecting the photoprotein's bioluminescence.

13. The method according to claim 12 wherein the specific cell lineage is the muscle heart cell lineage or the neuronal lineage.

14. The method according to claims 12 or 13 being performed by High Throughput Screening.

15. A method for identifying antagonists to a target, so that a variation of intracellular $Ca^{++}$ is obtained, comprising the steps of:

    a) providing a cell derivative according to claims 10 or 11;
    b) getting expression of said target;
    c) loading cells with a suitable chromophore as substrate;
    d) contacting cells with a compound library comprising putative antagonists for said target;
    e) contacting cells with a ligand able to stimulate said target;
    f) detecting the photoprotein's bioluminescence variation.

16. The method according to claim 15 wherein the specific cell lineage is the muscle heart cell lineage or the neuronal lineage or the mesenchymal cell lineage or the haematopoietic cell lineage.

**17.** The method according to claims 15 or 16 being performed by High Throughput Screening.

**18.** A method for identifying an agent stimulating the differentiation of cell derivatives according to claims 15 or 16 towards a specific cell lineage comprising the steps of:

a) providing a stem cell derivative of a non human transgenic animal according to claims 1 to 9 at an undifferentiated stage;
b) exposing said stem cell derivative to a compound library comprising putative inducing differentiation agents;
c) getting expression of at least one specific cell lineage target;
d) loading cells with a suitable chromophore as substrate;
e) stimulating said specific cell lineage target by a ligand so that a variation of intracellular $Ca^{++}$ is obtained;
f) detecting photoprotein's bioluminescence.

**19.** The method according to claim 18 being performed by High Throughput Screening.

**20.** A method for identifying an agent inhibiting the differentiation of stem cells derivatives according to claims 10 or 11 towards a specific cell lineage comprising the steps of:

a) providing stem cell derivatives of a non human transgenic animal according to claims 1 to 9 at an undifferentiated stage;
b) exposing said stem cell derivatives to a compound library comprising putative inhibiting differentiation agents;
c) exposing said stem cell derivatives to a known inducing differentiation agent to get expression of at least one specific cell lineage target;
d) loading cells with a suitable chromophore as substrate;
e) stimulating said specific cell lineage target by a ligand so that a variation of intracellular $Ca^{++}$ is obtained;
f) detecting photoprotein's bio luminescence.

**21.** The method according to claim 20 being performed by High Throughput Screening.

**22.** Use of a cell derivative of the non human transgenic animal according to any of claims 1 to 9 as positive controls for the "primary-like" cells obtained after differentiation of ES / photoprotein cells.

**23.** Use of a cell derivative of the non human transgenic animal according to any of claims 1 to 9 wherein said cell derivative containing a photoprotein as reporter system is used for screening of compounds.

**24.** Use of tissue or organ derivatives of the non human transgenic animal according to any of claims 1 to 9, containing a photoprotein as reporter system, for pharmacological *ex-vivo* studies or for transplantation experiments in animal models.

**25.** Use of the non human transgenic animal and the progeny thereof according to any of claims 1 to 9 for bio-imaging studies.

**26.** Use of the non human transgenic animal and the progeny thereof according to any of claims 1 to 9 for crossing with pharmacological relevant animal models.

**27.** Use of the non human transgenic animal and the progeny thereof according to claim 25 wherein the animal model is a transgenic animal carrying reporter genes, or KO or KI mutant animal models.

**28.** Use of the non human transgenic animal according to claims 1-9 for *in vivo* testing of toxicity and/or teratology of a substance.

**Patentansprüche**

**1.** Nichtmenschliches transgenes Tier, dessen Nachkommenschaft und Zellderivate, die in der Lage sind, ein Apophotoproteingen zu exprimieren und ein biolumineszentes Signal als Reaktion auf eine intrazelluläre Änderung der Calciumkonzentration zu erzeugen, wobei die Expression des Apophotoproteingens und/oder die Erzeugung eines biolumineszenten Signals als Reaktion auf eine intrazelluläre Änderung der Calciumkonzentration ubiquitär ist.

2. Nichtmenschliches transgenes Tier, dessen Nachkommenschaft und Zellderivate nach Anspruch 1, wobei die ubiquitäre Expression des Apophotoproteingens und/oder die Erzeugung eines biolumineszenten Signals als Reaktion auf eine intrazelluläre Änderung der Calciumkonzentration induzierbar ist.

3. Nichtmenschliches transgenes Tier, dessen Nachkommenschaft und Zellderivate nach Anspruch 1 oder 2, wobei das Apophotoproteingen aus der Gruppe, die folgendes umfasst, ausgewählt ist:

- Apophotoprotein-Sequenz von SEQ ID No. 1,
- Apophotoprotein-Sequenz der Clytin-Sequenz (GenBank-Aufnahmenummer Q08121), mutagenisiert in der folgenden Position Gly142->Cys, oder
- Apophotoprotein-Sequenz der Clytin-Sequenz (GenBank-Aufnahmenummer Q08121), mutagenisiert in den folgenden 12 Positionen: Gry58->Glu, Asp69->Val, Ala70->Cys, Lys76->Arg, Lys77->Gly, Ile78->Cys, Asp81->Glu, Va186->Ile, Glu87->Ala, Ala90->Gln, Val92->Leu, und Glu97->Gln.

4. Nichtmenschliches transgenes Tier, dessen Nachkommenschaft und Zellderivate nach einem der vorherigen Ansprüche, das ein nichtmenschliches Säugetier ist.

5. Nichtmenschliches transgenes Tier, dessen Nachkommenschaft und Zellderivate nach Anspruch 4, das ein Nagetier ist.

6. Nichtmenschliches transgenes Tier, dessen Nachkommenschaft und Zellderivate nach Anspruch 5, das eine Maus ist.

7. Nichtmenschliches transgenes Tier, dessen Nachkommenschaft und Zellderivate nach einem der vorherigen Ansprüche, wobei das Apophotoproteingen ein Apophotoproteingen von natürlichem oder rekombinantem oder synthetischem Ursprung ist.

8. Nichtmenschliches transgenes Tier, dessen Nachkommenschaft und Zellderivate nach Anspruch 7, wobei das Apophotoprotein eine natürliche oder mutagenisierte Mutante ist, die eine verbesserte Lumineszenzaktivität und/oder Calciumempfindlichkeit besitzt.

9. Nichtmenschliches transgenes Tier, dessen Nachkommenschaft und Zellderivate nach Anspruch 8, wobei das Apophotoproteingen eine Sequenz hat, die für die Verwendung von Säugetiercodons optimiert und/oder zu mitochondrialen Zielsequenzen verschmolzen ist.

10. Zellderivat des nichtmenschlichen transgenen Tieres nach einem der vorherigen Ansprüche, das in der Lage ist, sich in eine spezifische Zelllinie zu differenzieren, um die Expression eines spezifischen Zielgens zu erhalten.

11. Zellderivat des nichtmenschlichen transgenen Tieres nach Anspruch 10, wobei die spezifische Zelllinie die Herzmuskelzelllinie oder die neuronale Zelllinie oder die mesenchymale Zelllinie oder die hämatopoetische Zelllinie ist.

12. Verfahren zum Identifizieren eines Liganden, der ein spezifisches Ziel stimulieren kann, so dass eine Änderung des intrazellulären Ca++ erhalten ist, welches die folgenden Schritte umfasst:

a) Bereitstellen eines Zellderivats nach Anspruch 10 oder 11;
b) Erhalten einer Expression des Ziels;
c) Laden von Zellen mit einem geeigneten Chromophor als Substrat;
d) Kontaktieren der Zellen mit einer Substanzbibliothek, die mutmaßliche Liganden für das Ziel umfasst;
e) Feststellen der Biolumineszenz des Photoproteins.

13. Verfahren nach Anspruch 12, wobei die spezifische Zelllinie die Herzmuskelzelllinie oder die neuronale Zelllinie ist.

14. Verfahren nach Anspruch 12 oder 13, das durch Hochdurchsatztests ausgeführt ist.

15. Verfahren zum Identifizieren von Antagonisten eines Ziels, so dass eine Variation des intrazellulären Ca++ erhalten ist, welches die folgenden Schritte umfasst:

a) Bereitstellen eines Zellderivats nach den Ansprüchen 10 oder 11;

b) Erhalten einer Expression des Ziels;

c) Laden von Zellen mit einem geeigneten Chromophor als Substrat;

d) Kontaktieren der Zellen mit einer Substanzbibliothek, die mutmaßliche Antagonisten für das Ziel umfasst;

e) Kontaktieren der Zellen mit einem Liganden, der das Ziel stimulieren kann;

f) Feststellen der Variation der Biolumineszenz des Photoproteins.

16. Verfahren nach Anspruch 15, wobei die spezifische Zelllinie die Herzmuskelzelllinie oder die neuronale Zelllinie oder die mesenchymale Zelllinie oder die hämatopoetische Zelllinie ist.

17. Verfahren nach Anspruch 15 oder 16, das durch Hochdurchsatztests ausgeführt ist.

18. Verfahren zum Feststellen eines Mittels, das die Differenzierung von Zellderivaten stimuliert, nach Anspruch 15 oder 16, für eine spezifische Zelllinie, welches die folgenden Schritte umfasst:

a) Bereitstellen eines Stammzellenderivats eines nichtmenschlichen transgenen Tieres nach Anspruch 1 bis 9 in einem undifferenzierten Stadium;

b) Einwirkenlassen einer Stammzellbibliothek, die mutmaßliche Mittel zur Induzierung der Differenzierung umfasst, auf das Stammzellenderivat;

c) Erhalten einer Expression von mindestens einem spezifischen Zelllinienziel;

d) Laden von Zellen mit einem geeigneten Chromophor als Substrat;

e) Stimulieren des spezifischen Zelllinienziels durch einen Liganden, so dass eine Änderung des intrazellulären Ca++ erhalten ist;

f) Feststellen der Biolumineszenz des Photoproteins.

19. Verfahren nach Anspruch 18, das durch Hochdurchsatztests ausgeführt ist.

20. Verfahren zum Feststellen eines Mittels, das die Differenzierung von Stammzellderivaten hemmt, nach Anspruch 10 oder 11, für eine spezifische Zelllinie, welches die folgenden Schritte umfasst:

a) Bereitstellen von Stammzellderivaten eines nichtmenschlichen transgenen Tieres nach Anspruch 1 bis 9 in einem undifferenzierten Stadium;

b) Einwirkenlassen einer Substanzbibliothek, die mutmaßliche Mittel zur Hemmung der Differenzierung umfasst, auf die Stammzellderivate;

c) Einwirkenlassen eines bekannten Mittels zum Induzieren der Differenzierung auf die Stammzellderivate, um eine Expression von mindestens einem spezifischen Zelllinienziel zu erhalten;

d) Laden von Zellen mit einem geeigneten Chromophor als Substrat;

e) Stimulieren des spezifischen Zelllinienziels durch einen Liganden, so dass eine Änderung des intrazellulären Ca++ erhalten ist;

f) Feststellen der Biolumineszenz des Photoproteins.

21. Verfahren nach Anspruch 20, das durch Hochdurchsatztests ausgeführt ist.

22. Verwendung eines Zellderivats des nichtmenschlichen transgenen Tieres nach einem der Ansprüche 1 bis 9 als positive Kontrollen für die "primärähnlichen" Zellen, die nach Differenzierung von ES/Photoproteinzellen erhalten sind.

23. Verwendung eines Zellderivats des nichtmenschlichen transgenen Tieres nach einem der Ansprüche 1 bis 9, wobei das Zellderivat, das ein Photoprotein als Reportersystem enthält, zum Testen von Verbindungen verwendet wird.

24. Verwendung von Gewebs- oder Organderivaten des nichtmenschlichen transgenen Tieres nach einem der Ansprüche 1 bis 9, die ein Photoprotein als Reportersystem enthalten, für pharmakologische ex vivo-Untersuchungen oder für Transplantationsexperimente an Tiermodellen.

25. Verwendung des nichtmenschlichen transgenen Tieres und dessen Nachkommenschaft nach einem der Ansprüche 1 bis 9 für bildgebende Untersuchungen.

26. Verwendung des nichtmenschlichen transgenen Tieres und dessen Nachkommenschaft nach einem der Ansprüche 1 bis 9 zum Kreuzen mit pharmakologisch relevanten Tiermodellen.

27. Verwendung des nichtmenschlichen transgenen Tieres und dessen Nachkommenschaft nach Anspruch 25, wobei das Tiermodell ein transgenes Tier ist, welches Reportergene trägt, oder KO- oder KI-mutante Tiermodelle sind.

28. Verwendung des nichtmenschlichen transgenen Tieres nach Anspruch 1-9 zur in vivo-Untersuchung der Toxizität und/oder Teratologie einer Substanz.

**Revendications**

1. Animal transgénique non humain, sa progéniture et ses dérivés cellulaires, capables d'exprimer un gène d'une apophotoprotéine et de produire un signal bioluminescent en réponse à une variation de la concentration en calcium intracellulaire, l'expression du gène de l'apophotoprotéine et/ou la production d'un signal bioluminescent en réponse à la variation de la concentration en calcium intracellulaire étant ubiquitaire.

2. Animal transgénique non humain, sa progéniture et ses dérivés cellulaires selon la revendication 1, l'expression ubiquitaire du gène de l'apophotoprotéine et/ou la production d'un signal bioluminescent en réponse à la variation de la concentration en calcium intracellulaire étant inductible.

3. Animal transgénique non humain, sa progéniture et ses dérivés cellulaires selon la revendication 1 ou 2, le gène de l'apophotoprotéine étant choisi dans le groupe comprenant :

   - la séquence de l'apophotoprotéine de SEQ ID NO : 1,
   - la séquence de l'apophotoprotéine de la séquence de la Clytine (numéro d'accès GenBank Q08121) mutagénéisée à la position suivante Gly142 -> Cys, ou
   - la séquence de l'apophotoprotéine de la séquence de la Clytine (numéro d'accès GenBank Q08121) mutagénéisée aux 12 positions suivantes : Gly58->Glu, Asp69->Val, Ala70->Cys, Lys76->Arg, Lys77->Gly, Ile78->Cys, Asp81->Glu, Va186->Ile, Glu87->Ala, Ala90->Gln, Va192->Leu, et Glu97->Gln.

4. Animal transgénique non humain, sa progéniture et ses dérivés cellulaires selon l'une quelconque des revendications précédentes, étant un mammifère non humain.

5. Animal transgénique non humain, sa progéniture et ses dérivés cellulaires selon la revendication 4, étant un rongeur.

6. Animal transgénique non humain, sa progéniture et ses dérivés cellulaires selon la revendication 5, étant une souris.

7. Animal transgénique non humain, sa progéniture et ses dérivés cellulaires selon l'une quelconque des revendications précédentes, le gène de l'apophotoprotéine étant tout gène d'une apophotoprotéine, d'origine naturelle, recombinante ou synthétique.

8. Animal transgénique non humain, sa progéniture et ses dérivés cellulaires selon la revendication 7, l'apophotoprotéine étant un mutant naturel ou mutagénéisé ayant une activité luminescente et/ou une sensibilité au calcium améliorées.

9. Animal transgénique non humain, sa progéniture et ses dérivés cellulaires selon la revendication 8, le gène de l'apophotoprotéine ayant une séquence optimisée pour l'usage de codons de mammifères et/ou fusionnée à des séquences cibles mitochondriales.

10. Dérivé cellulaire de l'animal transgénique non humain selon l'une quelconque des revendications précédentes, capable de, ou différencié en une lignée cellulaire spécifique pour, obtenir l'expression d'un gène cible spécifique.

11. Dérivé cellulaire de l'animal transgénique non humain selon la revendication 10, la lignée cellulaire spécifique étant la lignée de cellules du muscle cardiaque ou la lignée neuronale ou la lignée cellulaire mésenchymateuse ou la lignée cellulaire hématopoïétique.

12. Procédé d'identification d'un ligand capable de stimuler une cible spécifique de manière à obtenir une variation du $Ca^{++}$ intracellulaire, qui comprend les étapes consistant à :

   a) fournir un dérivé cellulaire selon les revendications 10 ou 11 ;

EP 1 973 937 B1

b) obtenir l'expression de ladite cible ;
c) charger les cellules avec un chromophore adapté en tant que substrat ;
d) mettre les cellules en contact avec une banque de composés comprenant des ligands putatifs pour ladite cible ;
e) détecter la bioluminescence de la photoprotéine.

13. Procédé selon la revendication 12, la lignée cellulaire spécifique étant la lignée de cellules du muscle cardiaque ou la lignée neuronale.

14. Procédé selon les revendications 12 ou 13, étant réalisé par un criblage à haut débit.

15. Procédé d'identification des antagonistes d'une cible, de manière à obtenir une variation du Ca$^{++}$ intracellulaire, qui comprend les étapes consistant à :

a) fournir un dérivé cellulaire selon les revendications 10 ou 11 ;
b) obtenir l'expression de ladite cible ;
c) charger les cellules avec un chromophore adapté en tant que substrat ;
d) mettre les cellules en contact avec une banque de composés comprenant des antagonistes putatifs pour ladite cible ;
e) mettre les cellules en contact avec un ligand capable de stimuler ladite cible ;
f) détecter la variation de bioluminescence de la photoprotéine.

16. Procédé selon la revendication 15, la lignée cellulaire spécifique étant la lignée de cellules du muscle cardiaque ou la lignée neuronale ou la lignée cellulaire mésenchymateuse ou la lignée cellulaire hématopoïétique.

17. Procédé selon les revendications 15 ou 16, étant réalisé par un criblage à haut débit.

18. Procédé d'identification d'un agent stimulant la différenciation de dérivés cellulaires selon les revendications 15 ou 16 en une lignée cellulaire spécifique qui comprend les étapes consistant à :

a) fournir un dérivé de cellule souche d'un animal transgénique non humain selon les revendications 1 à 9 à un stade non différencié ;
b) exposer ledit dérivé de cellule souche à une banque de composés comprenant des agents induisant la différenciation putatifs ;
c) obtenir l'expression d'au moins une lignée cellulaire spécifique cible ;
d) charger les cellules avec un chromophore adapté en tant que substrat ;
e) stimuler ladite lignée cellulaire spécifique cible avec un ligand de manière à obtenir une variation du Ca$^{++}$ intracellulaire ;
f) détecter la bioluminescence de la photoprotéine.

19. Procédé selon la revendication 18, étant réalisé par un criblage à haut débit.

20. Procédé d'identification d'un agent inhibant la différenciation de dérivés de cellules souches selon les revendications 10 ou 11 en une lignée cellulaire spécifique, qui comprend les étapes consistant à :

a) fournir des dérivés de cellules souches d'un animal transgénique non humain selon les revendications 1 à 9 à un stade non différencié ;
b) exposer lesdits dérivés de cellules souches à une banque de composés comprenant des agents inhibant la différenciation putatifs ;
c) exposer lesdits dérivés de cellules souches à un agent induisant la différenciation connu pour obtenir l'expression d'au moins une lignée cellulaire spécifique cible ;
d) charger les cellules avec un chromophore adapté en tant que substrat ;
e) stimuler ladite lignée cellulaire spécifique cible avec un ligand de manière à obtenir une variation du Ca$^{++}$ intracellulaire ;
f) détecter la bioluminescence de la photoprotéine.

21. Procédé selon la revendication 20, étant réalisé par un criblage à haut débit.

22. Utilisation d'un dérivé cellulaire de l'animal transgénique non humain selon l'une quelconque des revendications 1 à 9, en tant que témoins positifs pour les cellules « de type primaire » obtenues après la différenciation des cellules ES/de photoprotéine.

23. Utilisation d'un dérivé cellulaire de l'animal transgénique non humain selon l'une quelconque des revendications 1 à 9, ledit dérivé cellulaire contenant une photoprotéine en tant que système rapporteur étant utilisé pour le criblage de composés.

24. Utilisation de dérivés de tissus ou d'organes de l'animal transgénique non humain selon l'une quelconque des revendications 1 à 9, contenant une photoprotéine en tant que système rapporteur, pour des études pharmacologiques *ex vivo* ou pour des expériences de transplantation sur des modèles animaux.

25. Utilisation de l'animal transgénique non humain et de sa progéniture selon l'une quelconque des revendications 1 à 9, pour des études de bio-imagerie.

26. Utilisation de l'animal transgénique non humain et de sa progéniture selon l'une quelconque des revendications 1 à 9, pour un croisement avec des modèles animaux pharmaceutiquement pertinents.

27. Utilisation de l'animal transgénique non humain et de sa progéniture selon la revendication 25, le modèle animal étant un animal transgénique portant des gènes rapporteurs, ou des modèles animaux mutants KO ou KI.

28. Utilisation de l'animal transgénique non humain selon les revendications 1 à 9, pour tester *in vivo* la toxicité et/ou la tératologie d'une substance.

**FIG. 1A**

ES / mito c-Photina® clones
96 MTP
100 µM Histamine

**FIG. 1B**

ES / mito c-Photina® clones
96 MTP
Total light release upon cell lysis

FIG. 2A

ES / mito c-Photina® 12 best clones
96 MTP – 20000 cells /well
100 µM Histamine

FIG. 2B

ES / mito c-Photina® 12 best clones
96 MTP
Total light release upon cell lysis

**FIG. 3A**

ES / mito c-Photina® 2 final clones
96 MTP – 10000 cells /well
100 µM Histamine

**FIG. 3B**

ES / mito c-Photina® 2 final clones
96 MTP – 20000 cells /well
100 µM Histamine

**Figure 4A**

**Figure 4B**

Figure 5

40X

**Figure 6A**

**Figure 6B**

**Figure 6C**

**Figure 7A**

Alpha actinin      20X

**Figure 7B**

MHC      10X

**Figure 7C**

GATA4      10X

**Figure 8A**

Cardiomyocytes-derived from ES / mito c-Photina® / 29 clone
96 MTP – 20000 cells /well
GqPCR Stimulation

Legend:
— Tyrode buffer
— 100 µM Norepinephrine
— 50 nM Endothelin-1

t (sec)

**Figure 8B**

Cardiomyocytes-derived from ES / mito c-Photina® / 29 clone
96 MTP – 20000 cells /well
L-type Calcium Channel Stimulation

Legend:
— 40 mM KCl
— 40 mM KCl + 5 µM Nifedipine

t (sec)

**Figure 8C**

Cardiomyocytes-derived from ES / mito c-Photina® / 29 clone
96 MTP – 20000 c/w
Total light release upon cell lysis

Day 9

**Figure 9A**

EB: Day 7          10X

**Figure 9B**

EB: Day 9          10X

**Figure 9C**

EB: Day 11          10X

**Figure 9D**

**Figure 10A and B**

Neurofilament H — 20X

NeuN — 20X

**Figure 10C**

Glial Fibrillary Acidic Protein — 10X

**Figure 10D**

Nestin — 20X

**Figure 11A**

Neurons-derived from ES / mito c-Photina® / 29 clone
Differentiation Day 14
96 MTP

**Figure 11B**

Neurons-derived from ES / mito c-Photina® / 29 clone
Differentiation Day 14
384 MTP

**Figure 12**

Neurons derived from ES / mito c-Photina® / 29 clone
384 MTP - Differentiation Day 16
40 mM KCl

## Figure 13A

Transgenic mito c-Photina® mice
96 MTP
Total light release upon cell lysis and
Injection of CaCl₂

## Figure 13B

Transgenic mito c-Photina® mice
Comparison of different experiment performed on mice of different ages.
Total light release upon cell lysis and injection of CaCl₂

## Figure 14A

Transgenic mito c-Photina® mice
Injection in the tail vein of 373 μM Coelenterazine (2.8 mg/kg)
in 300 μl of physiologic sol.
Total light release upon cell lysis and injection of CaCl$_2$

## Figure 14B

Transgenic mito c-Photina® mice
Further incubation of 3 h with coelenterazine
Total light release upon cell lysis and injection of CaCl$_2$

**Figure 15A**

mito c-Photina® transgenic mouse
96 MTP – 10 pancreatic islets /well
11 mM Glucidic Stimulus

**Figure 15B**

mito c-Photina® transgenic mouse
96 MTP – 10 pancreatic islets /well
40 mM KCl Depolarization Stimulus

**Figure 15C**

mito c-Photina® transgenic mouse
96 MTP – 10 pancreatic islets /well
Total light release upon cell lysis

**Fig. 16**

Macrophages derived from mito c-Photina®
transgenic mice
96 MTP - 20000 cells / well
Total light release upon cell lysis

**Fig. 17A**

ES / mito i-Photina® / 70 clone
96 MTP – 10000 cells /well
Histamine Dose Response

**Fig. 17B**

ES / mito i-Photina® 2 final clones
96 MTP – 10000 cells /well
Total light release upon cell lysis

Fig. 18A

ES / i-Photina® / 113 clone
96 MTP – 10000 cells /well
Histamine Dose Response

Fig. 18B

ES / i-Photina® 2 final clones
96 MTP – 10000 cells /well
Total light release upon cell lysis

**·FIG. 19A**

P19 / mito c-Photina® 1A1 clone
96 MTP – 20000 cells /well
Histamine response

**FIG. 19B**

P19 / mito c-Photina® 1A2 clone
96 MTP – 20000 cells /well
Histamine response

**FIG. 19C**

P19 / mito c-Photina® 2 final clones
96 MTP
Total light release upon cell lysis

**FIG. 20A**

NF H  40X

**FIG. 20B**

NeuN  20X

visible  20X

**FIG. 20C**

nestin  20X

visible  20X

**FIG. 21A**

Neurons derived from P19 / mito c-Photina® / 1A1 clone
384 MTP - Differentiation Day 8

**FIG. 21B**

Neurons derived from P19 / mito c-Photina® / 1A1 clone
384 MTP - Differentiation Day 11

FIG. 22

**Neurons derived from P19 / mito c-Photina® / 1A1 clone
384 MTP - Differentiation Day 8**

40 mM KCl

**FIG. 23A**

Undifferentiated P19 / mito c-Photina® / 1A1 clone
384 MTP – 3000 cells/well at 24 after seeding

100 µM Histamine

100 µM Glutamate

**FIG. 23B**

Neurons derived from P19 / mito c-Photina® / 1A1 clone
384 MTP - Differentiation Day 8

100 µM Histamine

100 µM Glutamate

**FIG. 24A**

P19 / mito photoprotein transient transfection
96 MTP – 5000 cells /well
100 µM Histamine

**FIG. 24B**

P19 / mito photoprotein transient transfection
96 MTP – 5000 cells /well
Total light release upon cell lysis

FIG. 25A

FIG. 25B

**FIG. 25C**

P19 / mito photoprotein pool stable transfection
96 MTP – 10000 and 20000 cells /well
Total light release upon cell lysis

**FIG. 26A**

P19 / mito c-Photina® 1A1 clone
384 MTP - 20000 cells/well

**FIG. 26B**

P19 / mito c-Photina® 1A2 clone
384 MTP - 20000 cells/well

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1413584 A
- WO 2006094805 A
- EP 05005390 A
- EP 06000171 A

**Non-patent literature cited in the description**

- **Burdon T. ; Smith A. ; Savatier P.** Signalling, cell cycle and pluripotency in embryonic stem cells. *Trends Cell Biol.,* 2002, vol. 12 (9), 432-8
- **Odorico J.S. ; Kaufman D.S. ; Thomson JA.** *Stem Cells. Multilineage differentiation from human embryonic stem cell lines,* 2001, vol. 19 (3), 193-204
- **Wobus, AM ; Boheler, KR.** Embryonic stem cells: prospects for developmental biology and cell therapy. *Physiological Reviews,* 2005, vol. 85, 635-678
- **Spangrude, G. J. ; Heimfeld, S. ; Weissman, I. L.** Purification and characterization of mouse hematopoietic stem cells. *Science,* 1988, vol. 241, 58-62
- **Krause D.S. ; Theise N.D. ; Collector M.I. ; Henegariu O. ; Hwang S. ; Gardner R. ; Neutzel S. ; Sharkis S.J.** Multi-organ, multi-lineage engraftment by a single bone marrow derived stem cell. *Cell,* 2001, vol. 105, 369-377
- **Reya T. ; Sean J. ; Morrison S.J. ; Clarke M.F.3 ; Weissman I.L.** Stem cells, cancer, and cancer stem cells. *Nature,* 2001, vol. 414, 105-111
- **Wagers A.J ; Weissman I.L.** Plasticity of adult stem cells. *Cell,* 2004, vol. 116 (5), 639-648
- **Baksh D. ; Song L. ; Tuan R.S.** Adult mesenchymal stem cells: characterization, differentiation, and application in cell and gene therapy. *J Cell Mol Med.,* 2004, vol. 8 (3), 301-316
- **Cai J. ; Weiss M.L. ; Rao MS.** In search of ''stemness''. *Exp Hematol.,* 2004, vol. 32 (7), 585-598
- **Donovan P.J. ; Gearhart J.** The end of the beginning for pluripotent stem cells. *Nature,* 2001, vol. 414, 92-97
- **Evans M.J. ; Kaufman M.H.** Establishment in culture of pluripotential cells from mouse embryos. *Nature,* 1981, vol. 292 (5819), 154-156
- **Martin G.R.** Isolation of a pluripotent cell line from early mouse embryos cultured in medium conditioned by teratocarcinoma stem cells. *Proc Natl Acad Sci U S A.,* 1981, vol. 78 (12), 7634-7638

- **Thomson J.A. ; Itskovitz-Eldor J. ; Shapiro S.S. ; Waknitz M.A. ; Swiergiel J.J. ; Marshall V.S. ; Jones J.M.** Embryonic stem cell lines derived from human blastocysts. *Science,* 1998, vol. 282 (5391), 1145-1147
- **Stevens, L. C.** The biology of teratomas. *Adv. Morphogen.,* 1967, vol. 6, 1-31
- **Gearhart J.D. ; Mintz B.** Contact-mediated myogenesis and increased acetylcholinesterase activity in primary cultures of mouse teratocarcinoma cells. *Proc Natl Acad Sci U S A.,* vol. 71 (5), 1734-1738
- **Kahan B.W. ; Ephrussi B.J.** Developmental potentialities of clonal in vitro cultures of mouse testicular teratoma. *Natl Cancer Inst.,* 1970, vol. 44 (5), 1015-1036
- **Jiang, L. I. ; Nadeau, J. H.** 129/Sv mice--a model system for studying germ cell biology and testicular cancer. *Mammal. Genome,* 2001, vol. 12, 89-94
- **McBurney M.W.** P19 embryonal carcinoma cells. *Int J Dev Biol.,* March 1993, vol. 37 (1), 135-140
- **Matsui, Y. ; Zsebo, K. ; Hogan, B. L.** Derivation of pluripotential embryonic stem cells from murine primordial germ cells in culture. *Cell,* 1992, vol. 70, 841-847
- **Labosky P.A. ; Barlow D.P. ; Hogan B.L.** Embryonic germ cell lines and their derivation from mouse primordial germ cells. *Ciba Found Symp.,* 1994, vol. 182, 157-68168-178
- **Labosky P.A. ; Barlow D.P. ; Hogan B.L.** Mouse embryonic germ (EG) cell lines: transmission through the germline and differences in the methylation imprint of insulin-like growth factor 2 receptor (Igf2r) gene compared with embryonic stem (ES) cell lines. *Development,* 1994, vol. 120 (11), 3197-3204
- **Resnick JL ; Bixler LS ; Cheng L ; Donovan PJ.** Long-term proliferation of mouse primordial germ cells in culture. *Nature,* 1992, vol. 359 (6395), 550-551

- **Bradley A. ; Evans M. ; Kaufman M.H. ; Robertson E.** Formation of germ-line chimeras from embryo-derived teratocarcinoma cell lines. *Nature,* 1984, vol. 309, 255-256
- **Stewart C.L. ; Gadi I. ; Bhatt H.** Stem cells from primordial germ cells can reenter the germ line. *Dev. Biol.,* 1994, vol. 161, 626-628
- **Brinster, R.L.** The effect of cells transferred into the mouse blastocyst on subsequent development. *J. Exp. Med.,* 1974, vol. 140, 1049-1056
- **Illmensee K. ; Mintz B.** Totipotency and normal differentiation of single teratocarcinoma cells cloned by injection into blastocysts. *Proc. Natl Acad. Sci. USA,* 1976, vol. 73, 549-553
- **Papaioannou V.E. ; Gardner R.L. ; McBurney M.W. ; Babinet, C. ; Evans M.J.** Participation of cultured teratocarcinoma cells in mouse embryogenesis. *J. Embryol. Exp. Morphol.,* 1978, vol. 44, 93-104
- **Martin G.R.** Teratocarcinomas and mammalian embryogenesis. *Science,* 1980, vol. 209, 768-776
- **McNeish J.** Embryonic stem cells in drug discovery. *Nat Rev Drug Discov.,* 2004, vol. 3 (1), 70-80
- **Seiler A. ; Visan A. ; Buesen R. ; Genschow E. ; Spielmann H.** Improvement of an in vitro Stem Cell Assay for developmental toxicity: the use of molecular endpoints in the embryonic stem cell test. *Reproductive Toxicology,* 2004, vol. 18, 231-240
- **Genschow E. ; Spielmann H. ; Scholz G. ; Seiler A. ; Brown N. ; Piersma A. ; Brady M. ; Clemann N. ; Huuskonen H. ; Paillard F.** The ECVAM international validation study on in vitro embryotoxicity tests: results of the definitive phase and evaluation of prediction models. European Centre for the Validation of Alternative Methods. *Altern Lab Anim.,* 2002, vol. 30 (2), 151-76
- **Genschow E. ; Spielmann H. ; Scholz G. ; Pohl I. ; Seiler A. ; Clemann N. ; Bremer S. ; Becker K.** Validation of the embryonic stem cell test in the international ECVAM validation study on three in vitro embryotoxicity tests. *Altern Lab Anim.,* 2004, vol. 32 (3), 209-244
- **Kendall J.M. ; Badminton M.N.** Aequorea victoria bioluminescence moves into an exciting new era. *Trends Biotechnology,* 1998, vol. 16 (5), 216-224
- Application of the photoprotein obelin to the measurement of free Ca++ in cells. **Campbell A.K. ; Hallet, R.A. ; Daw, M.E. ; Ryall, R.C. ; Hart ; Herring P.J.** Bioluminescence and Chemiluminescence, basic Chemistry and Analytical applications. Academy Press, 1981, 601-607
- **Herring P.J.** Some features of the bioluminescence of the radiolarian Thalassicola sp. *Mar. Biol.,* 1979, vol. 53, 213-216
- **Shimomura O. ; Johnson F.H. ; Saiga Y.** Extraction, purification and properties of aequorin, a bioluminescent protein from the luminous hydromedusan, Aequorea. *J. Cell. Comp. Physiol.,* 1962, vol. 59, 223-239
- **Shimomura O. ; Johnson F.H. ; Saiga, Y.** Further data on the bioluminescent protein, aequorin. *J. Cell. Comp. Physiol.,* 1963, vol. 62, 1-8
- **Morin J.G. ; Hastings J.W.** Biochemistry of the bioluminescence of colonial hydroids and other coelenterates. *J. Cell. Physiol.,* 1971, vol. 77, 305-311
- **Shimomura O. ; Johnson F.H. ; Saiga, Y.** Extraction and properties of halistaurin, a bioluminescent protein from the hydromedusan Halistaura. *J. Cell. Physiol.,* 1963, vol. 62, 9-15
- **Shimomura O. ; Shimomura A.** Halistaurin, phialidin and modified forms of aequorin as Ca++ indicator in biological systems. *Biochem. J.,* 1985, vol. 228, 745-749
- **Levine L.D. ; Ward W.W.** Isolation and characterization of a photoprotein "phialidin" and a spectrally unique green-fluorescent protein from the bioluminescent jellyfish Phialidium gregarium. Comp. *Biochem. Physiol.,* 1982, vol. 72B, 77-85
- **Morin J.G. ; Hastings J.W.** Energy transfer in a bioluminescent system. *J. Cell. Physiol.,* 1971, vol. 77, 313-318
- **Campbell A.K.** Extraction, partial purification and properties of obelin the calcium-activated protein from the hydroid Obelia geniculata. *Biochem.J.,* 1974, vol. 143, 411-418
- **Ward W.W. ; Selinger H.H.** Extraction and purification of calcium-activated photoprotein from the ctenophores Mnemiopsis sp. and Bern ovata. *Biochemistry,* vol. 13, 1491-1499
- **Ward W.W. ; Seliger H.H.** Properties of mnemiopsin, and berovin, calcium-activated photoproteins from the ctenophores Mnemiopsis sp. and Beroë ovata. *Biochemistry,* 1974, vol. 13, 1500-1510
- **Johnson F.H. ; Shimomura O.** Introduction to the bioluminescence of medusae, with special reference to the photoprotein aequorin. *Methods Enzymol.,* 1978, vol. 57, 271-291
- **Illarionov B.A. ; Bondar V.S. ; Illarionova V.A. ; Vysotski E.S.** Sequence of the cDNA encoding the Ca++-activated photoprotein obelin from the hydroid polyp Obelia longissima. *Gene,* 1995, vol. 153 (2), 273-274
- **Blinks J.R. ; Weir W.G. ; Hess P. ; Prendergast F.G.** Measurement of Ca++ concentrations in living cells. *Prog. Biophys. Mol. Biol.,* 1982, vol. 40, 1-114

- **Markova S.V. ; Vysotski E.S. ; Blinks J.R. ; Burakova L.P. ; Wang B.C. ; Lee J.** Obelin from the bioluminescent marine hydroid Obelia geniculata: cloning, expression, and comparison of some properties with those of other Ca2+-regulated photoproteins. *Biochemistry,* 2002, vol. 41 (7), 2227-36
- **Inouye S. ; Tsuji F.I.** Cloning and sequence analysis of cDNA for the Ca(2+)-activated photoprotein, clytin. *FEBS Lett.,* 1993, vol. 315 (3), 343-346
- **Tsuji F.I. ; Ohmiya Y. ; Fagan T.F. ; Toh H. ; Inouye S.** Molecular evolution of the Ca(2+)-binding photoproteins of the Hydrozoa. *Photochem Photobiol.,* 1995, vol. 62 (4), 657-661
- **Rizzuto R. ; Simpson A.W.M. ; Brini M. ; Pozzan T.** Rapid changes of mitochondrial Ca2+ revealed by specifically targeted recombinant aequorin. *Nature,* 1992, vol. 358, 325-328
- **Rizzuto R. ; Brini M. ; Murgia M. ; Pozzan T.** Microdomains with high Ca2+ close to IP3-sensitive channels that are sensed by neighbouring mitochondria. *Science,* 1993, vol. 262, 744-747
- **Rizzuto R. ; Bastianutto C. ; Brini M ; Murgia M. ; Pozzan T.** Mitochondrial Ca2+ homeostasis in intact cells. *J. Cell Biol.,* 1994, vol. 126, 1183-1194
- Manipulating the mouse embryo. **Nagy A. ; Gertsenstein M. ; Vinterstein K. ; Behringer R.** A laboratory manual. Cold Spring Harbor Laboratory Press, 2003
- Embryonic Stem Cells. Methods and protocols. **Turksen K.** Methods in Molecular Biology. Humana Press, 2002, vol. 185
- **Boheler K.R.** ES cell differentiation to the cardiac lineage. *Methods in enzymology,* 2003, vol. 365, 228-241
- **Bloemers S.M. ; Leurs R. ; Smit M.J. ; Verheule S. ; Tertoolen L.G.J. ; Timmerman H. ; de Laat S.W.** Mouse P19 embryonal carcinoma cells express functional Histamine H1-receptors. *Biochemical and Biophysical Research' Communications,* 1993, vol. 191, 118-125
- **Sambrook J. ; Russel D. W.** Molecular cloning. A laboratory manual. Cold Spring Harbor Laboratory Press, 2001
- **Ramakers C. ; Ruijter J.M. ; Deprez R.H. ; Moorman A.F.** Assumption-free analysis of quantitative real-time polymerase chain reaction (PCR) data. *Neuroscience Letters,* 2003, vol. 339, 62-66
- Cell culture methods and induction of differentiation of embryonal carcinoma cell lines. **Rudnicki M.A. ; McBurney M.W.** Teratocarcinomas and Embryonic Stem Cells: A Practical Approach. IRL Press, 1987, 19-49
- **J. Paquin B. ; Danalache M. ; Jankowski S.M. ; McCann J. ; Gutkowska J.** Oxytocin induces differentiation of P19 embryonic stem cells to cardiomyocytes. *Proc. Nat. Acad. Sci. USA,* 2002, vol. 99, 9550-9555
- **Bolino A. ; Bolis A. ; Previtali S.C. ; Dina G. ; Bussini S. ; Dati G. ; Amadio S. ; Del Carro U. ; Mruk D.D. ; Feltri M.L.** Disruption of Mtmr2 produces CMT4B1-like neuropathy with myelin outfolding and impaired spermatogenesis. *J Cell Biol,* 2004, vol. 167, 711-721
- **Bain G. ; Kitchens D. ; Yao M. ; Huettner J.E. ; Gottlieb D.I.** Embryonic stem cells express neuronal properties in vitro. *Dev Biol,* 1995, vol. 168, 342-357
- **Okada Y. ; Shimazaki T. ; Sobue G. ; Okano H.** Retinoic-acid-concentration dependent acquisition of neural cell identity during in vitro differentiation of mouse embryonic stem cells. *Dev Biol,* 2004, vol. 275, 124-142
- **Bhaumik S. ; Gambhir S.S.** Optical imaging of Renilla luciferase reporter gene expression in living mice. *PNAS,* 2002, vol. 99 (1), 377-82
- **Maltais D. ; Desroches D. ; Aouffen M. ; Mateescu M.A. ; Wang R. ; Paquin J.** The blue copper ceruloplasmin induces aggregation of newly differentiated neurons: a potential modulator of nervous system organization. *Neuroscience,* 2003, vol. 121 (1), 73-82
- Isolation and culture of murine macrophages Meth. in Mol. Biol. **Davies J. Q. ; Gordon S.** Basic Cell Culture Protocols. 2005, vol. 290, 91-103

SEQUENCE LISTING

<110> AxxAM s.r.l.

<120> LUMINESCENT TRANSGENIC NON HUMAN ANIMALS, PROGENY, CELL DERIVATIVES AND USES THEREOF

<130> PCT 96693

<160> 8

<170> PatentIn Version 3.3

<210> 1
<211> 195
<212> PRT
<213> chimeric protein

<400> 1

```
Met Ser Ser Lys Tyr Ala Val Lys Leu Lys Thr Asp Phe Asp Asn Pro
1               5                   10              15

Arg Trp Ile Lys Arg His Lys His Met Phe Asp Phe Leu Asp Ile Asn
            20                  25                  30

Gly Asn Gly Lys Ile Thr Leu Asp Glu Ile Val Ser Lys Ala Ser Asp
        35                  40                  45

Asp Ile Cys Ala Lys Leu Gly Ala Thr Pro Glu Gln Thr Lys Arg His
        50                  55                  60

Gln Asp Ala Val Glu Ala Phe Phe Lys Lys Ile Gly Met Asp Tyr Gly
65                  70                  75                  80

Lys Glu Val Glu Phe Pro Ala Phe Val Asp Gly Trp Lys Glu Leu Ala
                85                  90                  95

Thr Ser Glu Leu Lys Lys Trp Ala Arg Asn Glu Pro Thr Leu Ile Arg
                100                 105                 110

Glu Trp Gly Asp Ala Val Phe Asp Ile Phe Asp Lys Asp Gly Ser Gly
        115                 120                 125

Thr Ile Thr Leu Asp Glu Trp Lys Ala Tyr Gly Lys Ile Ser Gly Ile
    130                 135                 140

Ser Pro Ser Gln Glu Asp Cys Glu Ala Thr Phe Arg His Cys Asp Leu
145                 150                 155                 160

Asp Asn Ser Gly Asp Leu Asp Val Asp Glu Met Thr Arg Gln His Leu
                165                 170                 175

Gly Phe Trp Tyr Thr Leu Asp Pro Glu Ala Asp Gly Leu Tyr Gly Asn
            180                 185                 190


Gly Val Pro
        195
```

<210> 2
<211> 99
<212> DNA
<213> Homo sapiens

<400> 2


atgtccgtcc tgacgccgct gctgctgcgg ggcttgacag gctcggcccg gcggctccca       60

gtgccgcgcg ccaagatcca ttcgttggga tccgccacc                              99


<210> 3
<211> 19
<212> DNA
<213> synthetic primer

<400> 3
caccaagtgt gcgtggagg      19

<210> 4
<211> 20
<212> DNA
<213> synthetic primer

<400> 4
gcgatctcct tgccgtactc      20

<210> 5
<211> 20
<212> DNA
<213> synthetic primer

<400> 5
cacgtactcg gatggaagcc      20

<210> 6
<211> 20
<212> DNA
<213> synthetic primer

<400> 6
ccctgatgct cttcgtccag      20

<210> 7
<211> 20
<212> DNA
<213> synthetic primer

<400> 7
ggaggtgatt cagccacagc      20

<210> 8
<211> 20
<212> DNA
<213> synthetic primer

<400> 8
tcggcttctg atgcgtctta      20